# EUROPEAN PATENT APPLICATION

(11) **EP 4 741 414 A1**
(43) Date of publication of application: **13.05.2026**
(21) Application number: 24836016.6
(22) Date of filing: 01.07.2024
(51) Int. Cl.: C07K 7/08, A61K 31/7088, A61K 45/00, A61K 47/64, C07K 7/06, C07K 7/64, C07K 14/00, C12N 15/87, C12Q 1/02, C40B 40/08

(54) **PEPTIDE HAVING CELL MEMBRANE PERMEABILITY AND SCREENING METHOD THEREFOR**

(30) Priority: 03.07.2023 JP 2023109588
(71) Applicant: Mescue-Janusys Inc., Tokyo 103-0023 (JP)
(72) Inventor: HARADA Mitsunori, Tokyo 103-0023 (JP); KITAMURA Koichiro, Tokyo 103-0023 (JP)
(74) Representative: Müller-Boré & Partner Patentanwälte PartG mbB
(86) International application number: PCT/JP2024/023780
(87) International publication number: WO 2025/009503

(57) **Abstract**

Provided is a peptide or a salt thereof having plasma membrane-penetrating activity, the peptide comprising an amino acid sequence X that satisfies the following conditions (1) to (4): (1) the amino acid sequence X includes at least one amino acid residue selected from R, K, and H, and the total number of these three amino acid residues is 1 to 7; (2) the amino acid sequence X includes at least one amino acid residue selected from Y, W, and F, and the total number of these three amino acid residues is 1 to 5; (3) the amino acid sequence X includes at least one amino acid residue selected from L, V, and M, and the total number of these three amino acid residues is 1 to 4; and (4) the amino acid sequence X has 6 to 15 amino acid residues. Also provided is a method for screening a peptide having plasma membrane-penetrating activity, comprising the steps of: (A) preparing a cDNA display library comprising cDNA display molecules; (B) contacting cells with the cDNA display library and incubating; and (C) recovering the cDNA display molecules from intracellular contents of the cells or from a permeate through the cells.

## Description

### Technical Field

The present invention relates to peptides having plasma membrane-penetrating activity and methods for screening such peptides.

### Background Art

In recent years, a molecularly targeted agent that acts on a specific protein or gene serving as a target has been attracting attention, and hence a protein and a gene present in cells are each one of attractive potential drug targets. As a method of delivering a drug into a cell, there has been known a method including bonding a peptide having plasma membrane-penetrating activity (hereinafter also referred to as "cell-penetrating peptide") to impart the cell-penetrating activity to a drug or a carrier thereof (e.g., Patent Literature 1).

### Citation List

### Patent Literature

[PTL 1] JP 2021-531274 A

### Summary of Invention

### Technical Problem

The number of cell-penetrating peptides, which exhibit high intracellular delivery efficiency and hence have been actually applied to clinical uses, is small, and hence there has been a demand for a novel cell-penetrating peptide. In addition, while existing cell-penetrating peptides are introduced into a wide variety of cell types; however, they are generally considered to lack cell selectivity. Accordingly, a cell-type-selective cell-penetrating peptide has been desired for alleviating a side effect.

In view of the foregoing, a primary object of the present invention is to provide a novel peptide exhibiting high plasma membrane-penetrating activity, and another object thereof is to provide a novel cell-penetrating peptide that is cell-type-selective.

### Solution to Problem

[1] According to one aspect of the present invention, there is provided a peptide or a salt thereof having plasma membrane-penetrating activity, including an amino acid sequence X that satisfies the following conditions (1) to (4): (1) the amino acid sequence X includes at least one amino acid residue selected from R, K, and H, and the total number of these three amino acid residues is 1 to 7; (2) the amino acid sequence X includes at least one amino acid residue selected from Y, W, and F, and the total number of these three amino acid residues is 1 to 5; (3) the amino acid sequence X includes at least one amino acid residue selected from L, V, and M, and the total number of these three amino acid residues is 1 to 4; and (4) the amino acid sequence X has 6 to 15 amino acid residues.
[2] In the peptide or the salt thereof according to the above-mentioned item [1], the amino acid sequence X may be linear, and within the amino acid sequence X, the number of consecutive occurrences of D, F, L, Q, R, S, V, and Y may be 2 or less for each such residue, and the number of consecutive occurrences of the other amino acid residues may be 1; or the amino acid sequence X may form a cyclic structure, and within the amino acid sequence X, the number of consecutive occurrences of F, L, and Y may be 2 or less for each such residue, the number of consecutive occurrences of R may be 3 or less, and the number of consecutive occurrences of the other amino acid residues may be 1.
[3] In the peptide or the salt thereof according to the above-mentioned item [1] or [2], the amino acid sequence X may be linear, and a proportion of the total number of eight amino acid residues E, M, A, L, Q, K, R, and H to the total number of amino acid residues constituting the amino acid sequence X may be 25% to 60%; or the amino acid sequence X may form a cyclic structure via a disulfide bond between two cysteine residues, and a proportion of the total number of eight amino acid residues E, M, A, L, Q, K, R, and H to the total number of amino acid residues constituting the amino acid sequence X excluding the two cysteine residues may be 25% to 60%.
[4] The peptide or the salt thereof according to any one of the above-mentioned items [1] to [3] may exhibit cell-type-selective plasma membrane-penetrating activity.
[5] The peptide or the salt thereof according to any one of the above-mentioned items [1] to [4] may exhibit selective plasma membrane-penetrating activity toward at least one selected from the group consisting of macrophage cells, cancer cells, neutrophils, mast cells, T cells, B cells, gastrointestinal epithelial cells, the blood-brain barrier, and skin tissue.
[6] In the peptide or the salt thereof according to any one of the above-mentioned items [1] to [5], the number of amino acid residues may be 6 to 30.
[7] The peptide or the salt thereof according to any one of the above-mentioned items [1] to [6] may have the GRAVY value of from -2.6 to 1.9, and may be linear.
[8] The peptide or the salt thereof according to any one of the above-mentioned items [1] to [7] may include an amino acid sequence represented by any one of SEQ ID NOs: 1-40 or a homologous sequence thereof, wherein the homologous sequence may include 1 to 5 amino acid residue substitutions, insertions, or deletions in the amino acid sequence represented by any one of SEQ ID NOs: 1-40 and may have a sequence identity of 60% or more to the amino acid sequence
[9] According to another aspect of the present invention, there is provided a drug-peptide conjugate including a drug and the peptide or the salt thereof according to any one of the above-mentioned items [1] to [8], wherein the peptide or the salt thereof is bonded to the drug.
[10] In the drug-peptide conjugate according to the above-mentioned item [9], the drug may be a protein or a nucleic acid.
[11] According to another aspect of the present invention, there is provided a use of the peptide or the salt thereof of any one of the above-mentioned items [1] to [8] for drug delivery.
[12] According to another aspect of the present invention, there is provided a method for screening a peptide having plasma membrane-penetrating activity, including the steps of: (A) preparing a cDNA display library including cDNA display molecules; (B) contacting cells with the cDNA display library and incubating the resulting mixture; and (C) recovering the cDNA display molecules from intracellular contents of the cells or from a permeate through the cells.
[13] The screening method according to the above-mentioned item [12] may further include preparing a sub-cDNA display library using the recovered cDNA display molecules.
[14] In the screening method according to the above-mentioned item [12] or [13], a selection including the steps (A), (B), and (C) in this order may be repeated twice or more.

### Advantageous Effects of Invention

According to an embodiment of the present invention, there is provided the novel cell-penetrating peptide. In addition, according to the method for screening a cell-penetrating peptide according to the embodiment of the present invention, the novel cell-penetrating peptide can be suitably screened for a plurality of cell types by using a cDNA display method.

### Brief Description of Drawings

FIGS. **1** are each a schematic view for describing an example of a method of producing a cDNA display library.
FIG. **2** is a schematic view for describing the structure of an example of a cDNA display molecule.
FIG. **3** is a schematic view for describing a screening method according to one embodiment of the present invention.
FIG. **4** is a schematic view for describing the screening method according to one embodiment of the present invention.
FIG. **5** is a view for illustrating a template DNA sequence for protein expression in a cell-free system.
FIG. **6** is a view for illustrating the template DNA sequence for the protein expression in the cell-free system.
FIG. **7** is a view for illustrating the template DNA sequence for the protein expression in the cell-free system.
FIG. **8** is a view for illustrating the template DNA sequence for the protein expression in the cell-free system.
FIG. **9** is a view for illustrating the template DNA sequence for the protein expression in the cell-free system.
FIG. **10** is a set of photographs showing the results of the intracellular delivery assay of the eGFP-peptide fusion protein.
FIG. **11** is a set of photographs showing the results of the intracellular delivery assay of the eGFP-peptide fusion protein.
FIG. **12** is a set of photographs showing the results of the intracellular delivery assay of the IgG-peptide conjugates in macrophage cells.
FIG. **13** is a graph showing the ratio of the intracellularly delivered amount of the IgG-peptide conjugate to that of the IgG in the macrophage cells.
FIG. **14** is a graph showing the ratio of the intracellularly delivered amount of the IgG-peptide conjugate to that of the IgG in the macrophage cells.
FIG. **15** is a graph showing the ratio of the intracellularly delivered amount of the IgG-peptide conjugate to that of the IgG in A549 cells.
FIG. **16** is a graph collectively showing the ratios of the intracellularly delivered amounts of the IgG-peptide conjugate to that of the IgG in the macrophage cells and the A549 cells.
FIG. **17** is a graph showing the luciferase activity-inhibiting effect of siRNA(LUC)-encapsulated lipid nanoparticles in A549-Luc cells.
FIG. **18** is a graph showing the luciferase activity-inhibiting effect of the siRNA(LUC)-encapsulated lipid nanoparticles in BxPC-3-Luc#2 cells.
FIG. **19** is a graph showing the luciferase-expressing effect of mRNA(LUC)-encapsulated lipid nanoparticles in the A549 cells.
FIG. **20** is a graph showing the luciferase-expressing effect of the mRNA (LUC)-encapsulated lipid nanoparticles in Panc-1 cells.

### Description of Embodiments

Preferred embodiments of the present invention are described below. However, the present invention is not limited to these embodiments. In addition, the respective embodiments may be appropriately combined unless the combination is inappropriate in the context. Further, the expression "from ... to ..." indicating a numerical range as used herein includes the upper limit and lower limit values of the numerical range.

Herein, amino acids or amino acid residues may be represented by the following one-letter codes: alanine (A), leucine (L), arginine (R), lysine (K), asparagine (N), methionine (M), aspartic acid (D), phenylalanine (F), cysteine (C), proline (P), glutamine (Q), serine (S), glutamic acid (E), threonine (T), glycine (G), tryptophan (W), histidine (H), tyrosine (Y), isoleucine (I), and valine (V).

### A. Peptide having Plasma Membrane-Penetrating Activity

According to one aspect of the present invention, there is provided a peptide or a salt thereof having plasma membrane-penetrating activity, the peptide including an amino acid sequence X that satisfies the following conditions (1) to (4):
(1) the amino acid sequence X includes at least one amino acid residue selected from R, K, and H, and the total number of these three amino acid residues is 1 to 7;
(2) the amino acid sequence X includes at least one amino acid residue selected from Y, W, and F, and the total number of these three amino acid residues is 1 to 5;
(3) the amino acid sequence X includes at least one amino acid residue selected from L, V, and M, and the total number of these three amino acid residues is 1 to 4; and
(4) the amino acid sequence X has 6 to 15 amino acid residues.

As described above, a peptide including an amino acid sequence X, which consists of 6 to 15 amino acid residues and contains, in the specified numbers, at least one basic amino acid residue selected from R, K, and H, at least one aromatic amino acid residue selected from Y, W, and F, and at least one amino acid residue having a hydrophobic linear side chain selected from L, V, and M, can exhibit high plasma membrane-penetrating activity.

In the amino acid sequence X satisfying the above-mentioned (1), the total number of the three amino acid residues R, K, and H may be from 1 to 6.

The ratio of the total number of the three amino acid residues R, K, and H to the total number of amino acid residues constituting the amino acid sequence X is, for example, from 7% to 54%, and may be from 7% to 42%.

The ratio of the total number of the three amino acid residues Y, W, and F to the total number of amino acid residues constituting the amino acid sequence X is, for example, from 7% to 50%, and may be from 8% to 50%.

The ratio of the total number of the three amino acid residues L, V, and M to the total number of amino acid residues constituting the amino acid sequence X may be, for example, from 7% to 40%.

In one embodiment, the amino acid sequence X may include at least one amino acid residue selected from K, L, and V.

The number of amino acid residues constituting the amino acid sequence X may be, for example, 7 to 15, and in another example, 8 to 14.

The above-mentioned cell-penetrating peptide may be linear or may have a cyclic structure. The cyclic structure may be formed by, for example, a bond between the N-terminal amino acid residue and C-terminal amino acid residue of the peptide, a bond between one of the terminal amino acid residues thereof and an amino acid residue in a non-terminal portion thereof, or a bond between amino acid residues in non-terminal portions thereof. The cyclic structure may be formed by, for example, a disulfide bond between two cysteine residues (C). For example, the amino acid sequence X may be linear or may form a cyclic structure.

In one embodiment, the amino acid sequence X is linear, and when amino acid residues selected from D, F, L, Q, R, S, V, and Y are present in the amino acid sequence X, the number of consecutive occurrences for each such residue may be no more than 2, and the number of consecutive occurrences of the other amino acid residues may be 1.

In another embodiment, the amino acid sequence X forms a cyclic structure, and when amino acid residues selected from F, L, and Y are present in the amino acid sequence X, the number of consecutive occurrences for each such residue may be no more than 2, the number of consecutive occurrences of R may be no more than 3, and the number of consecutive occurrences of the other amino acid residues may be 1.

As used herein, the number of consecutive occurrences for each amino acid residue refers to the number of that residue that may occur successively within the amino acid sequence X. Accordingly, when the number of consecutive occurrences is 1, the residue does not occur consecutively in the amino acid sequence X (i.e., no two such residues are adjacent).

In one embodiment, the amino acid sequence X may contain 25% to 60% of amino acid residues having a propensity to form an α-helix structure, selected from E, M, A, L, Q, K, R, and H (P. Y. Chou, G. D. Fasmann Adv. Enzymol. Relat. Areas Mol. Biol. 47, 45-148 (1978)). Herein, for a linear peptide, the proportion is defined as the ratio of the total number of amino acid residues with α-helical propensity to the total number of amino acid residues constituting the amino acid sequence X. For a cyclic peptide, the proportion is defined as the ratio of the total number of amino acid residues with α-helical propensity to the total number of amino acid residues constituting the amino acid sequence X excluding the two cysteine residues that form the disulfide bond. In this embodiment, the proportion is preferably from 30% to 50%.

In one embodiment (Embodiment "a"), the amino acid sequence X is linear, includes at least H as a basic amino acid residue, and satisfies the following (i) and/or (ii):
(i) at least one residue H is incorporated as two consecutive amino acid residues represented by HV, VH, HY, or YH; and
(ii) when H is located at the C-terminal, the amino acid sequence X includes at least one amino acid residue each of the six amino acids V, T, R, E, Y, and K.

In one embodiment (Embodiment "b"), the amino acid sequence X is linear, includes only R as a basic amino acid residue, and satisfies the following (iii) and/or (iv):
(iii) at least one residue R located at a position except both the terminals of the amino acid sequence X is incorporated as two consecutive amino acid residues represented by AR, VR, or RL; and
(iv) R located at the N-terminal thereof is incorporated as two consecutive amino acid residues represented by RV or RW.

In one embodiment (Embodiment "c"), the amino acid sequence X is linear, includes only K as a basic amino acid residue, and includes at least two amino acid residues selected from F, V, L, and I so that the ratio of the total number of the residues to the total number of amino acid residues constituting the amino acid sequence X may be from 40% to 50%.

In one embodiment (Embodiment "d"), the amino acid sequence X is linear, and while the sequence does not include H as a basic amino acid residue, the sequence includes one or more each of residues K and R, and fourth and eighth amino acid residues in a direction from its N-terminal to its C-terminal are each K or R.

In one embodiment, the cell-penetrating peptide (e.g., the amino acid sequence X) has a grand average of hydropathy (GRAVY) value of from -2.6 to 1.9. The GRAVY value is an indicator of the hydrophilicity or hydrophobicity of the entirety of a peptide or a protein calculated from its amino acid composition, and when the value is positive, the peptide or the protein tends to be hydrophobic. A peptide (e.g., a linear peptide), which has a GRAVY value in the above-mentioned range while including at least one basic amino acid residue selected from R, K, and H, at least one aromatic amino acid residue selected from Y, W, and F, and at least one kind of amino acid residue having a hydrophobic liner side chain, the residue being selected from L, V, and M, may have excellent plasma membrane-penetrating activity. The GRAVY value of the cell-penetrating peptide (e.g., the amino acid sequence X) is preferably from - 2.6 to 0.7, and may be, for example, from -2.6 to 0.3. The GRAVY value may be calculated in accordance with the method of Kyte et al. (J. Kyte, RF. Doolittle, J Mol. Biol 152 (1982) 105). When the amino acid sequence X is a linear sequence including 10 or more amino acid residues, the GRAVY value of any appropriate 10 consecutive amino acid residues is preferably 1.0 or less, more preferably 0.7 or less, still more preferably 0.3 or less.

In one embodiment, the cell-penetrating peptide preferably includes an amino acid residue having a functional group in a side chain thereof, and more preferably includes, at its N-terminal and/or its C-terminal, the amino acid residue having a functional group in a side chain thereof. Such cell-penetrating peptide can suitably bind to, for example, a drug (e.g., a protein or a nucleic acid) and a carrier thereof through a reaction utilizing the functional group. Examples of the amino acid residue having a functional group in a side chain thereof include cysteine, serine, threonine, tyrosine, aspartic acid, glutamic acid, lysine, and arginine. Of those, cysteine, aspartic acid, glutamic acid, and lysine are preferred.

The cells in which the cell-penetrating peptide exhibits plasma membrane-penetrating activity are not particularly limited and may be any cell type. Examples thereof include: cancer cells; immune cells, such as macrophage cells, neutrophil cells, T cells, and B cells; mast cells; cells or a cell group for forming skin tissues, such as epidermis and dermis; gastrointestinal epithelial cells; vascular epithelial cells; and cells or a cell group for forming the blood-brain barrier.

In one embodiment, the above-mentioned cell-penetrating peptide has cell-type-selective penetrating activity. Specifically, the peptide described herein may exhibit higher plasma membrane-penetrating activity in a predetermined cell (typically, the cell type used for screening) than in other cell types. For example, in the screening method as described below, a peptide selected by screening using a lung cancer cell line may exhibit higher plasma membrane-penetrating activity in that cell line than in other cell types. In addition, a peptide selected by screening using a macrophage cell line exhibits higher plasma membrane-penetrating activity in that cell line than in other cell types. As used herein, plasma membrane-penetrating activity may be determined as follows: a peptide is labeled with a fluorescent dye, such as an Alexa Fluor fluorescent dye (Molecular Probes), a Cy fluorescent dye, or FITC, or a fusion protein of the peptide with eGFP or GFP is prepared, the labeled peptide or the fusion protein is incubated with cells at a concentration of 5 µM for 1 hour, and intracellular fluorescence attributable to the labeled peptide or the fusion protein is observed by fluorescence microscopy, preferably, higher fluorescence intensity than that of a control to which no peptide is appended is observed. However, the determination of plasma membrane-penetrating activity is not limited to this method. In one embodiment, a cell-penetrating peptide, when bonded to a drug, can enhance the intracellular delivery of the drug.

In the above-described cell-penetrating peptide, one or more amino acid residues may be added to the N-terminal and/or C-terminal of the amino acid sequence X. For example, for purposes such as imparting reactivity toward a drug or a carrier therefor, introducing a linker, modulating hydrophilicity or hydrophobicity, or facilitating purification, peptides in which desired amino acid residues are appended to the N- and/or C-terminal of the amino acid sequence X are also encompassed within the plasma membrane-penetrating peptides of the embodiment of the present invention, provided that they retain cell-penetrating activity. The number of the amino acid residues to be appended is not particularly limited, insofar as plasma membrane-penetrating activity is maintained. The number may be, for example, 1, 2, or 3 or more, and may be, for example, 24 or less, 15 or less, 10 or less, 8 or less, or 6 or less.

The lower limit of the number of the amino acid residues for constituting the above cell-penetrating peptide may be, for example, 6 or more, 7 or more, or 8 or more. The upper limit is not particularly limited; however, from the standpoint of ease of synthesis and the like, it may be, for example, 30 or less, 25 or less, 20 or less, or 15 or less.

The above-mentioned amino acid sequence X is specifically, for example, an amino acid sequence represented by any one of SEQ ID Nos: 1 to 40 shown in Table 1, or a homologous sequence thereof.

In one embodiment, the cell-penetrating peptide consists of an amino acid sequence represented by any one of SEQ ID NOs: 1 to 40.

In another embodiment, the cell-penetrating peptide consists of an amino acid sequence homologous to an amino acid sequence represented by any one of SEQ ID Nos: 1 to 40 (provided that the homologous sequence preferably satisfies one or more, more preferably all, of the requirements described for the amino acid sequence X, including items (1) to (4) above, the content ratios of basic amino acid residues, aromatic amino acid residues, and/or amino acid residues having a hydrophobic linear side chain, the number of consecutive occurrences of the predetermined amino acid residues, the content ratio of amino acid residues having a propensity to form an α-helix structure, and the predetermined GRAVY value range). The term "homologous sequence" as used herein means a sequence having an identity of, for example, 60% or more, preferably 70% or more, more preferably 80% or more, still more preferably 90% or more, still further more preferably 95% or more to the original amino acid sequence. In one embodiment, a homologous sequence may include one, two, three, four, or five substitutions, insertions, or deletions of amino acid residues relative to the original amino acid sequence; preferably one, two, or three; more preferably one or two. The substitutions may, for example, be conservative substitutions, which means replacing one amino acid residue with another amino acid residue having similar structural and/or chemical properties. By way of example, non-polar amino acids include G, A, L, I, V, P, W, and M; polar uncharged amino acids include S, T, C, Y, N, and Q; basic amino acids include R, K, and H; and acidic amino acids include D and E. In addition, for example, the substitutions may be carried out so that, in the resulting peptide sequence (i.e., the homologous sequence), the total proportion of residues having a propensity to form an α-helical structure (specifically, E, M, A, L, Q, K, R, and H) is 25% to 60%, preferably from 30% to 50%. Such substitutions may be carried out by replacing at least one amino acid residue selected from E, M, A, L, Q, K, R, and H with a residue selected from natural amino acids other than these and C (specifically, V, I, Y, W, F, T, G, N, P, S, and D), or by replacing at least one natural amino acid residue having a propensity to form an α-helical structure with another amino acid residue having a propensity to form an α-helical structure.

In still another embodiment, the above cell-penetrating peptide is a peptide in which one or more amino acid residues are appended to the N-terminal and/or C-terminal of an amino acid sequence represented by any one of SEQ ID Nos: 1 to 40, or a homologous sequence thereof. For example, for purposes such as imparting reactivity toward a drug or a carrier therefor, introducing a linker, modulating hydrophilicity or hydrophobicity, or facilitating purification, peptides in which desired residues are appended to the N-terminal and/or C-terminal of the amino acid sequence represented by any one of SEQ ID Nos: 1 to 40, or the homologous sequence thereof are also encompassed within the cell-penetrating peptides of the embodiments of the present invention, provided that they retain plasma membrane-penetrating activity. As described above, the number of appended residues may be, for example, 1, 2, or 3 or more, and may be, for example, 24 or less, 15 or less, 10 or less, 8 or less, or 6 or less.

**Table 1**

| Amino acid sequence | SEQ ID NO | Amino acid sequence | SEQ ID NO |
|---|---|---|---|
| CKMWSPYGQETYC | 1 | FSVRLPMFSFRL | 21 |
| CNRYRMIVDQSC | 2 | IQIKVMAWLT | 22 |
| CSKNWYGWMFC | 3 | KWPVINVEVK | 23 |
| FQIYKFAFMV | 4 | LFARLLDMFF | 24 |
| GWKIPYLWHV | 5 | LYARILNWIF | 25 |
| GYYKRMIKQQ | 6 | LYHTAIKTFT | 26 |
| LFKAWLGVYH | 7 | QALLKLYFSF | 27 |
| MNYHKSNRHN | 8 | RLIFRHVIDMIF | 28 |
| RVVSYLAEYH | 9 | VHVHVMINFF | 29 |
| VDMKSSNRLW | 10 | WYMVHLRSEW | 30 |
| YFDDWLAPYH | 11 | SHYSLIVFSR | 31 |
| CIRYYVARHVRRRC | 12 | CHMVNTYKWTFFIC | 32 |
| CRMAPQFNHVC | 13 | CTPWKNRNVLLWYC | 33 |
| RVVSYLAEYQ | 14 | HFHYYMHSVY | 34 |
| TVTRGERYKH | 15 | KILIFHYIFV | 35 |
| VHSWVDMAKN | 16 | RWLIFHYMLL | 36 |
| YVDDWLAPYH | 17 | RWPLWMWFVW | 37 |
| QYNMVRQDLI | 18 | RYYAHWLHYM | 38 |
| VRVKTYWENH | 19 | YYLLPLHVKH | 39 |
| FLPMFSRLGLQI | 20 | RWNCVSCAFL | 40 |

Among the above-mentioned amino acid sequences represented by SEQ ID NOs: 1 to 40, the amino acid sequences represented by SEQ ID NOs: 5, 7, 8, 9, 11, 15, 16, 17, 19, 26, 28, 29, 30, 31, 34, 35, 36, 38, and 39 each correspond to the amino acid sequence X according to Embodiment "a", the amino acid sequences represented by SEQ ID NOs: 14, 18, 20, 21, 24, 25, 37, and 40 each correspond to the amino acid sequence X according to Embodiment "b", the amino acid sequences represented by SEQ ID NOs: 4, 22, 23, and 27 each correspond to the amino acid sequence X according to Embodiment "c", and the amino acid sequences represented by SEQ ID NOs: 6 and 10 each correspond to the amino acid sequence X according to Embodiment "d". Specific examples of the amino acid sequence X that can suitably exhibit the effects of the present invention include the amino acid sequences represented by SEQ ID NOs: 8, 15, 19, 23, and 37, and homologous sequences thereof.

Examples of a method of producing the cell-penetrating peptide include a chemical synthesis method, such as a solid-phase synthesis method, a stepwise extension method, or a liquid-phase synthesis method, a fermentation method, and an enzymatic method. Of those, a solid-phase synthesis method is preferred. Examples of the solid-phase synthesis method include a Fmoc synthesis method and a Boc synthesis method.

The cell-penetrating peptide may be in the form of a derivative or a salt as long as the effects of the present invention are obtained. Accordingly, in this description, the peptide may include the form of a derivative of the peptide, or a salt of the peptide or the derivative thereof unless the form is clearly inappropriate in the context.

Examples of derivatives of the peptide include those in which one or more functional groups of the peptide-such as the N-terminal amino group, the C-terminal carboxyl group, and side-chain carboxyl, amino, guanidino, hydroxyl, or thiol groups -are substituted with various substituents. The substituent is not particularly limited, and examples thereof include an alkyl group, an acyl group, a hydroxy group, an amino group, an alkylamino group, a nitro group, an amide group, a sulfonyl group, a halogen, and various protective groups. Those substituents may each be further substituted with a halogen such as fluorine. In addition, the substitution may be performed by introducing a label, such as a fluorescent label or a biotin label.

The salt of the peptide is preferably a pharmacologically acceptable salt. Examples of the pharmacologically acceptable salt include an acid addition salt and a base addition salt. Examples of the acid addition salt include an inorganic acid salt and an organic acid salt. Examples of the inorganic acid salt include a hydrochloric acid salt, a hydrobromic acid salt, a sulfuric acid salt, a hydroiodic acid salt, a nitric acid salt, and a phosphoric acid salt. Examples of the organic acid salt include a citric acid salt, an oxalic acid salt, an acetic acid salt, a formic acid salt, a propionic acid salt, a benzoic acid salt, a trifluoroacetic acid salt, a maleic acid salt, a tartaric acid salt, a methanesulfonic acid salt, a benzenesulfonic acid salt, and a p-toluenesulfonic acid salt. Examples of the base addition salt include an inorganic base salt and an organic base salt. Examples of the inorganic base salt include a sodium salt, a potassium salt, a calcium salt, a magnesium salt, and an ammonium salt. Examples of the organic base salt include a triethylammonium salt, a triethanolammonium salt, a pyridinium salt, and a diisopropylammonium salt.

### B. Drug-peptide Conjugate

A drug-peptide conjugate according to embodiments of the present invention includes a drug and a cell-penetrating peptide as set forth in the item (A) bonded to the drug. By virtue of the peptide's plasma membrane-penetrating activity, the drug-peptide conjugate may itself exhibit plasma membrane-penetrating activity.

The drug is not particularly limited, and examples thereof include: a protein (e.g., an antibody or a functional fragment thereof, a hormone, or an enzyme); a nucleic acid (e.g., a high-molecular weight nucleic acid, such as a plasmid DNA or a mRNA, or a low-molecular weight nucleic acid, such as a siRNA, a miRNA, an antisense nucleic acid, or an aptamer); any other physiologically active substance (e.g., an antitumor agent, a signal transduction inhibitor, an antimetabolite, an analgesic agent, an anti-inflammatory agent, or an antimicrobial agent); a fluorescent dye; and a contrast agent. The weight (molecular weight) of the drug is not particularly limited. Specifically, the weight of the drug may be less than 500 Da, or 500 Da or more, and may be, for example, from 1,000 Da to 150,000 Da, or for example, from 3,000 Da to 150,000 Da. The cell-penetrating peptide according to the embodiment of the present invention can impart cell-penetrating activity even to a biopolymer, such as a protein or a nucleic acid.

The number of the molecules of the cell-penetrating peptide to be bonded to the drug may be appropriately set in accordance with, for example, the weight or molecular weight of the drug, or the cell-penetrating activity of the cell-penetrating peptide. The number of the molecules of the cell-penetrating peptide to be bonded to the drug is, for example, from 1 to 10 per one molecule of the drug, and may be from 1 to 5 or from 1 to 3.

In the drug-peptide conjugate, the drug and the cell-penetrating peptide may be directly bonded, or may be bonded through a linker. In the case of the direct bonding, the drug-peptide conjugate may be formed by causing a functional group of the drug and a functional group of the cell-penetrating peptide to react with each other. In the case of the bonding through the linker, the drug-peptide conjugate may be formed by, for example, causing a functional group positioned at one end of the linker and the functional group of the drug to react with each other, and causing a functional group positioned at the other end of the linker and the functional group of the cell-penetrating peptide to react with each other. For example, an alkylene group having 2 to 10 carbon atoms that may include an ether bond, an amide bond, an ester bond, or the like (e.g., a polyoxyethylene group) may be present between the functional groups positioned at both the terminals of the linker. When the drug is a protein, the drug-peptide conjugate may be obtained as a fusion protein in which the cell-penetrating peptide is linked to the N-terminal and/or C-terminal of the protein by a genetic engineering approach.

Examples of the combination of the functional groups in the above-mentioned reaction include: an azide group and an alkyne; a thiol group and a (meth)acryloyl group; a thiol group and a maleimide group; a thiol group and a thiol group; a thiol group and a carboxyl group; a (meth)acryloyl group and a hydroxyl group; a (meth)acryloyl group and an amino group; a carboxyl group and an amino group; a carboxyl group and a hydroxyl group; and an amino group and a hydroxyl group.

### C. Use of Peptide having Plasma membrane-Penetrating Activity

The cell-penetrating peptide described in the item (A) may be used for drug delivery. In one embodiment, the peptide is used as a targeting moiety for delivery of a drug to an intracellular target. In this case, it is preferable that the peptide exhibits selective cell-penetrating activity for the intended cell. In another embodiment, the peptide is used upon intravascular or oral administration of a drug to enhance translocation from the vasculature into tissues or translocation from the gastrointestinal tract into tissues. In this case, it is preferable that the peptide exhibits cell-penetrability toward vascular endothelial cells or gastrointestinal epithelial cells, more preferably with cell-type selectivity. In yet another embodiment, the peptide is used for delivery of a drug to the brain to improve penetration thereof into the brain; in this case, it is preferable that the peptide exhibits penetrating activity across the blood-brain barrier In a further embodiment, the peptide is used for transdermal delivery of a drug to enhance penetration thereof from the skin into the bloodstream; in this case, it is preferable that the peptide exhibits skin penetrating activity. Alternatively, where the drug is intended to act on the skin, it is preferable that the peptide exhibits intracellular delivery into epidermal or dermal cells.

In the use of the above-mentioned cell-penetrating peptide in the drug delivery, the cell-penetrating peptide may be bonded to the drug or a carrier thereof (specifically, a constituent of the carrier). Accordingly, according to the present invention, there may be provided a method of improving the cell-penetrating activity of a drug or a carrier for drug delivery, the method including bonding the above-mentioned cell-penetrating peptide to the drug, or the carrier for drug delivery or a constituent thereof, and a method of producing a drug or a carrier for drug delivery, which has cell-penetrating activity imparted thereto or is improved in cell-penetrating activity, the method including bonding the above-mentioned cell-penetrating peptide to a drug, or a carrier for drug delivery or a constituent thereof.

The above-mentioned bond may be a covalent bond, or may be a non-covalent bond. A conjugate in which the drug and the cell-penetrating peptide are covalently bonded is as described in the section B. The carrier to which the cell-penetrating peptide is bonded is not particularly limited. Preferred examples of the carrier of the drug include nanoparticles, such as a liposome, a micelle, and a vesicle. Those nanoparticles are generally composed of a hydrophobic compound or polymer, a hydrophilic compound or polymer, and/or an amphiphilic compound or polymer, each of which contains, for example, a polyamino acid, a lipid, a polysaccharide, or any other polymer (e.g., polyethylene glycol). In one embodiment, the cell-penetrating peptide may be bonded to an end of such constituent for forming each of the nanoparticles so that the peptide may be exposed to the surface of the nanoparticle. A method for the bonding may be appropriately selected in accordance with purposes. In the case of a covalent bond, the bonding may be performed in the same manner as in the bonding of the drug and the cell-penetrating peptide, and hence such a combination of functional groups that react with each other as described above may be utilized. In the case of a non-covalent bond, the bonding (complexation) may be performed by utilizing, for example, an electrostatic interaction, a hydrophobic interaction, a hydrogen bond, or a van der Waals force.

### D. Screening Method for Plasma Membrane-Penetrating Peptides

According to embodiments of the present invention, a method for screening peptides having plasma membrane-penetrating activity includes:
a step A of preparing a cDNA display library including cDNA display molecules;
a step B of contacting cells with the cDNA display library and incubating the resulting mixture; and
a step C of recovering the cDNA display molecules from the intracellular contents of the cells or from a permeate through the cells.

The above-mentioned screening method utilizes a cDNA display method. The cDNA display method is one form of genotype-phenotype linkage technology and enables a one-to-one (1:1) correspondence between the function and/or phenotype of a gene expression product (e.g., a protein) and the cDNA encoding the corresponding gene. Specifically, the cDNA display molecules recovered in the step (C) are subjected to PCR to amplify and identify the gene encoding the peptide borne by the molecule, thereby determining the amino acid sequence of the cell-penetrating peptide. Because the screening method utilizes highly stable cDNA display molecules, it can efficiently obtain desired peptides even in systems employing live cells. Moreover, by selecting the cells used for screening, peptides having cell-penetrating activity toward desired cell types can be efficiently obtained.

In the above-mentioned screening method, a sub-cDNA display library can be prepared using the recovered cDNA display molecules. In this case, the preparation of the sub-cDNA display library may be designated as step (A), followed by steps (B) and (C). That is, in the screening method, steps (A), (B), and (C) constitute one selection round; by performing step (A) again using the selection product (the recovered cDNA display molecules), the next selection round is initiated, and, as a result, the selection can be repeated two or more times. By carrying out two or more rounds of selection, peptides with relatively low cell-penetrating activity are eliminated, enabling efficient acquisition of peptides with higher cell-penetrating activity. The selection may be repeated until the cDNA sequences of the recovered cDNA display molecules sufficiently converge (e.g., until the read number of the most abundant sequence accounts for 0.1% or more, preferably 1% or more of the total read number). The number of selection rounds is, for example, 1 or more, preferably 2 or more, and may be 3 or more, or 4 or more; and is, for example, no more than 6, preferably no more than 5.

### D-1. Step A

In the step A, a cDNA display library including cDNA display molecules is prepared. Methods for preparing cDNA display libraries are known, and any appropriate method may be used.

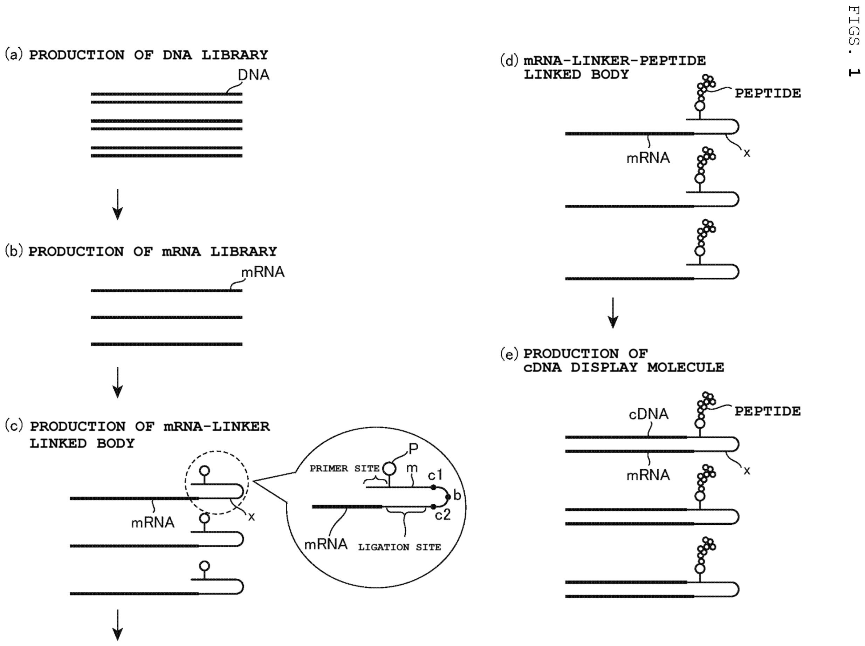

By way of example, as illustrated in FIG. 1, the cDNA display library can be prepared by a method including: providing a DNA library containing DNA that encodes random amino acid sequences (step "a"); transcribing the DNA of the DNA library into mRNA to obtain an mRNA library (step "b"); ligating to the 3'-terminal of the mRNA of the mRNA library a puromycin-bearing linker X to obtain mRNA-linker conjugates (step "c"); translating the mRNA-linker conjugate in a cell-free translation system and allowing the peptide thus produced to be coupled to the puromycin, thereby obtaining mRNA-linker-peptide conjugates (step "d"); and reverse-transcribing the mRNA of the mRNA-linker-peptide conjugate to generate cDNA, thereby obtaining cDNA display molecules (step "e").

In one embodiment, as illustrated in a main-part enlarged view of FIG. **1(c)****,** the linker **X** has puromycin **P** linked to its main skeleton **"m"** including a single-stranded DNA and/or a peptide nucleic acid (PNA), and has, at the 5'-terminal of the main skeleton, a ligation site that can be hybridized with the mRNA to be linked to the 3'-terminal of the mRNA, and the linker may have a reverse transcription primer site at the 3'-terminal of the main skeleton. When the linker has such structure, the linker can be efficiently linked to the mRNA to provide the mRNA-linker conjugate in the step "c". In addition, the reverse transcription from the reverse transcription primer site in the step "e" can reliably correlate the peptide and the cDNA.

The main skeleton has a length of preferably from 10 mer to 60 mer, more preferably from 10 mer to 45 mer, still more preferably from 15 mer to 30 mer.

Puromycin is a compound functioning as an analog of the 3'-terminal of an aminoacyl-tRNA, and can bind to a peptide chain extending in a ribosome. Puromycin may be the following puromycin-like compound: the compound has a structure similar to that of the 3'-terminal of the aminoacyl-tRNA; and when a protein is synthesized in a translation system, the compound has an ability to bind to the C-terminal of the synthesized protein.

The linker **X** preferably further has, in the main skeleton **"m",** a solid-phase binding site **"b"** that can bind to a solid phase site, and a pair of cleavage sites **c1** and **c2** arranged at positions sandwiching the above-mentioned solid-phase binding site **"b".** When the linker has such structure, the purification and reverse transcription of the mRNA (step "e") can be performed under a state in which the mRNA-linker-peptide conjugate obtained in the step "d" is bonded (immobilized) to a solid phase through the linker. When the linker is cleaved at the pair of cleavage sites **c1** and **c2** after the reverse transcription, the cDNA display molecules can be suitably prepared.

The solid-phase binding site only needs to be capable of bonding the mRNA-linker-peptide conjugate to the solid phase through the linker, and specific examples thereof include: a base to which biotin can be bonded (e.g., deoxythymidine (dT)); a base having bonded thereto biotin (e.g., biotin-deoxythymidine (biotin-dT)); a base modified with an amino group (e.g., an amino-modified deoxythymidine); a base modified with a carboxyl group (e.g., a carboxyl-modified deoxythymidine); and a base modified with a thiol group (e.g., a thiol-modified deoxythymidine). Of those, biotin-deoxythymidine is preferred from the viewpoint of an affinity for avidin.

The pair of cleavage sites is, for example, a pair of enzymatic cleavage sites. Thus, the mRNA-linker-peptide conjugate bonded to the solid phase by the solid-phase binding site can be cleaved with, for example, an enzyme that cleaves the above-mentioned cleavage sites, and hence the cDNA display molecules can be removed from the solid phase. The enzymatic cleavage sites may be appropriately selected in accordance with the kind of the enzyme to be used. The enzymatic cleavage sites are each specifically, for example, riboguanosine (riboG).

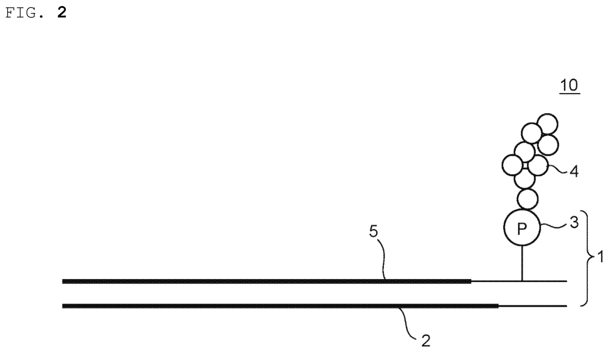

FIG. **2** is a schematic diagram illustrating an example structure of a cDNA display molecule. The cDNA display molecule **10** is a cleavage remnant of linker **X,** and includes: a linker residue **1** bearing puromycin **3;** cDNA **5** connected to one end of the linker residue **1;** mRNA **2** connected to the other end thereof and hybridized with the cDNA **5;** and a peptide **4** bound to the puromycin **3.**

For details about the structure of the cDNA display molecule **10** and a method of preparing the molecule, and the method of preparing the cDNA display library, reference may be made to, for example, WO 2006/041194 A1.

The diversity of the cDNA display library to be used in the first selection (diversity of random amino acid sequences presented in the library) may be, for example, from 1.0×10¹⁰ to 1.0×10¹³, preferably from 1.0×10¹¹ to 1.0×10¹³, more preferably from 1.0×10¹¹ to 1.0×10¹².

### D-2. Step B

In the step (B), cells are contacted with the cDNA display library and the resulting mixture is incubated. The cells to be contacted may be selected as appropriate for the purpose and may include a single cell type or two or more cell types. The cells may also be organized as a tissue. Accordingly, embodiments in which a tissue is contacted with the cDNA display library and incubated in the step (B) are likewise encompassed within the screening method of embodiments of the present invention.

The incubation temperature and the incubation time may each be appropriately set depending on the cells to be contacted, the intended use of the cell-penetrating peptide, and the like. For example, the incubation temperature may be set to the culture temperature of the cells. The incubation time may be, for example, from 5 minutes to 72 hours; in some embodiments, from 30 minutes to 24 hours, or from 10 minutes to 4 hours.

### D-3. Step C

In the step (C), the cDNA display molecules are recovered from the intracellular contents of the cells or from a permeate through the cells after the above-mentioned incubation. The recovery of the cDNA display molecules may be carried out using commercially available kits or the like.

Upon subjecting the recovered cDNA display molecules to PCR and identifying the cDNA nucleotide sequences and the encoded amino acid sequences, if sequence convergence is insufficient, a sub-cDNA display library is prepared using the PCR product (step (A)) and subjected to another round of selection. If sequence convergence is sufficient, the selection is terminated without performing the step (A), and a peptide consisting of the identified amino acid sequence is obtained as a cell-penetrating peptide.

The preparation of the sub-cDNA display library from the recovered cDNA display molecules may be carried out in the same manner as the method for preparing the cDNA display library in the step (A), except that an mRNA library is prepared by using the PCR product of the recovered cDNA display molecules as the DNA library.

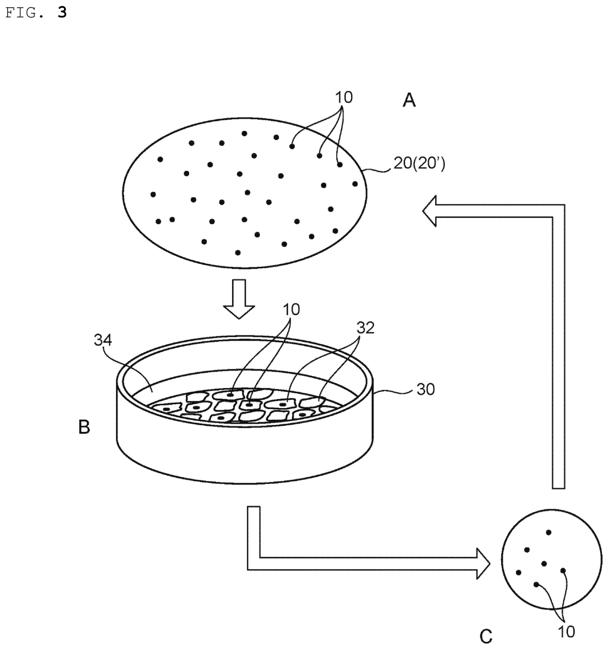

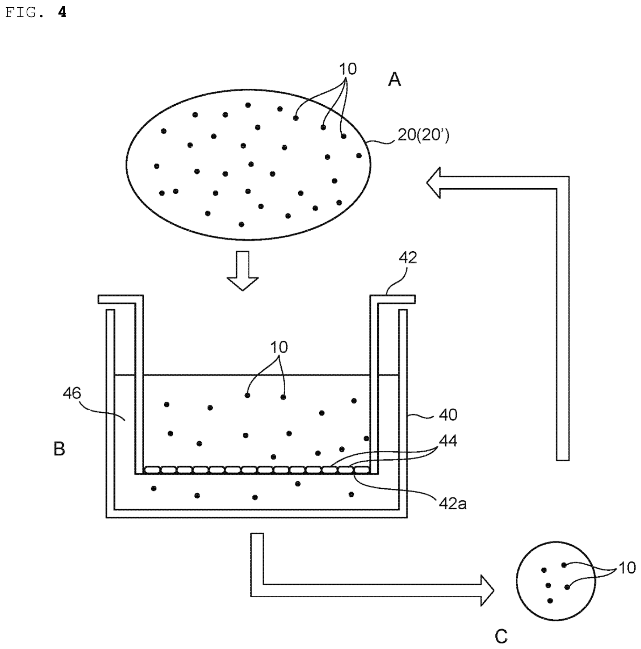

FIG. **3** and FIG. **4** are schematic diagrams each illustrating a screening method according to one embodiment of the present invention. In the embodiment illustrated in FIG. **3****,** the contacting and incubation in the step (B) between the cells and the cDNA display library are carried out by adding the cDNA display library **20** to a culture vessel **30** containing cells **32** and culture medium **34,** followed by incubation. The addition amount of the cDNA display library (more specifically, the addition amount of its cDNA display molecules) is, for example, from 100 fmol to 1,000 fmol, and may be preferably from 200 fmol to 400 fmol. In one embodiment, the addition amount is such that the number of cDNA display molecules per cell at the time of their presentation is, for example, from 1.0×10⁵ to 1.0×10⁸, preferably from 1.0×10⁶ to 1.0×10⁸, more preferably from 1.0×10⁷ to 1.0×10⁸. The incubation time may be set as appropriate for the assay purpose, and may be relatively short (e.g., from 5 minutes to 4 hours, preferably from 30 minutes to 2 hours), or may be relatively long (e.g., from 20 hours to 72 hours, preferably from 24 hours to 48 hours).

After incubation, the cells **32** are harvested and, after washing as necessary, the intracellular contents are recovered by hypotonic treatment, crushing treatment, or the like. Next, the cDNA display molecules **10** are recovered from the intracellular contents using a nucleic acid purification kit or the like (step C). The recovered cDNA display molecules **10** are subjected to PCR. When sequence convergence is insufficient, a sub-cDNA display library **20'** is prepared (step A), and the subsequent steps B and C are carried out using the sub-cDNA display library **20'.**

Any appropriate cells may be used as the above-mentioned cells in accordance with purposes. Examples thereof include: cancer cells; immune cells, such as macrophage cells, neutrophil cells, T cells, and B cells; mast cells; and cells or cell populations constituting skin tissues, such as epidermis and dermis. According to the embodiment illustrated in FIG. **3****,** peptides that traverse the plasma membrane and remain within cells (in other words, exhibit cytosolic translocation property) can be efficiently screened.

In the embodiment illustrated in FIG. **4****,** the contacting and incubation in step (B) between the cells and the cDNA display library are carried out by adding the cDNA display library **20** to a cell culture insert **42** inserted into a culture well **40** and containing cells **44** and culture medium **46,** followed by incubation. The bottom surface **42a** of the cell culture insert **42** is preferably confluent and preferably completely covered with a monolayer or multilayer of cells. The amount of the cDNA display library added (more specifically, the amount of cDNA display molecules added) may be, for example, from 100 fmol to 1,000 fmol, preferably from 200 fmol to 400 fmol. The incubation time may be set to, for example, from 5 minutes to 24 hours, preferably from 15 minutes to 4 hours, more preferably from 30 minutes to 2 hours.

Because the bottom surface 42a of the cell culture insert 42 is formed of a porous membrane, the culture medium 46 outside the insert 42 may contain a permeate that has passed from the inside to the outside of the insert through the cells 44. Accordingly, the cDNA display molecules **10** that have traversed the cells **44** can be recovered from the culture medium **46** outside the cell culture insert (step C). The cDNA display molecules **10** may be recovered from the culture medium using a nucleic acid purification kit or the like. The recovered cDNA display molecules **10** are subjected to PCR. When the identified amino acid sequences have not sufficiently converged, a sub-cDNA display library **20'** is prepared (step A), and the subsequent steps B and C are carried out using the sub-cDNA display library **20'.**

Alternatively, the following may be performed: the cDNA display library is added to the culture medium outside the cell culture insert, and the cDNA display molecules that have been contacted with cells through the porous membrane at the bottom surface of the insert and penetrated through the cells, are recovered from the culture medium inside the insert.

As the cells, any suitable cells may be used depending on the purpose. Examples thereof include: gastrointestinal epithelial cells; vascular epithelial cells; cells or cell populations constituting the blood-brain barrier; and cells or cell populations constituting skin tissues, such as epidermal or dermal cells. According to the embodiment illustrated in FIG. **4****,** peptides capable of traversing a cellular layer from one side to the other (e.g., peptides that can penetrate from the gastrointestinal tract into the bloodstream, from the bloodstream into tissues, or from the bloodstream into the brain, or can be percutaneously absorbed) can be efficiently screened.

### Examples

The present invention is specifically described below by way of Examples, but the present invention is not limited to these Examples.

### [Experimental Example 1: Production of cDNA Display Library]

A cDNA display library including a random peptide region was prepared by an in vitro virus method including utilizing a linker described in WO 2006/041194 A1. Details are as described below.

An mRNA library was prepared with RiboMAX Large Scale RNA Production System-SP6 Kit (Promega Corporation) on the basis of a DNA library including a DNA encoding a peptide including 8 to 15 random amino acid residues. 1 Picomole to 2 pmol of a cDNA, 4 µL of a rNTP mixture, 2 µL of a transcriptase, and 4 µL of a reaction buffer were mixed, and then the volume of the mixture was adjusted to 20 µL with purified water (the reagents except the cDNA were included in the kit). The resultant solution for transcription was incubated at 37°C for 2.5 hours, and then 1 µL of RQ1 RNase-Free DNase (1 U/1 µL) was added thereto, followed by incubation at 37°C for 20 minutes. After that, the mRNA was purified with RNeasy Mini Kit (QIAGEN) in accordance with a manual included in the kit.

Subsequently, a conjugate of the mRNA and a puromycin linker described in WO 2006/041194 A1 (hereinafter referred to as "linker") was prepared. Details are as described below.

A solution was prepared by mixing 20 pmol of the mRNA and 20 µM of the linker, and then adjusting the volume of the mixture to 20 µL with purified water (Ambion, Inc.). The solution was incubated at 90°C for 30 seconds, and was cooled to 70°C at 0.12°C/sec. After that, the solution was incubated for 30 seconds, and was cooled to 4°C at 0.06°C/sec. 1 Microliter of T4 polynucleotide kinase (Takara Bio Inc.), 1 µL of T4 RNA ligase (Takara Bio Inc.), 2 µL of T4 RNA ligase buffer (included in the T4 RNA ligase), and 1.5 µL of 0.1% BSA (included in the T4 RNA ligase) were loaded into the solution after the cooling, and the mixture was incubated at 25°C for 1 hour to prepare a mRNA-linker conjugate.

Next, a peptide was synthesized by mixing the mRNA-linker conjugate into a cell-free translation system. 20 Microliters of the conjugate, 50 µL of a wheat germ extract (Promega Corporation), 8 µL of an amino acid mixture (Promega Corporation), 5 µL of 1 M KOAc (Ambion, Inc.), 5 µL of a RNase inhibitor (Thermo Fisher Scientific, Inc.), and 12 µL of purified water were mixed to prepare a translation reaction liquid, and the liquid was incubated at 25°C for 15 minutes. After that, 46 µL of a salt mix (18.25 µL of 3 M KCl and 4.75 µL of 1 M MgCl₂) was added to the liquid, and the mixture was incubated at 25°C for 1 hour. 28 Microliters of 0.5 M EDTA having a pH of 8.0 (Thermo Fisher Scientific, Inc.) was further added to the incubated product. Thus, a mRNA-linker-peptide conjugate (post-translational product) was obtained.

Purification with DynaBeads MyOne Streptavidin C1 (Thermo Fisher Scientific, Inc.) (hereinafter referred to as "magnetic beads") was performed by utilizing biotin in the linker in the post-translational product. 150 Microliters of the magnetic beads were suspended well, and were then washed with a 2× binding buffer (buffer composition: 20 mM Tris-HCl (pH: 7.5), 2 M NaCl, 2 mM EDTA, and 0.2% polyoxyethylene (20) sorbitan monolaurate) whose amount was the same as that of the beads twice (the beads were added and suspended into the buffer, and then the supernatant was discarded). Subsequently, 348 µL of the post-translational product was added to the washed product, and the mixture was incubated at 25°C for 30 minutes while being rotated with a rotator. After that, the supernatant was discarded, and the residue was washed with 400 µL of a 1x binding buffer twice. The washed product was further washed with 200 µL of a 1x RT buffer (Promega Corporation) once.

8 Microliters of a 2.5 mM dNTP mixture (Takara Bio Inc.), 4 µL of an RNase inhibitor (Thermo Fisher Scientific, Inc.), 20 µL of a 10× RT buffer (Promega Corporation), and 166 µL of purified water were added to the magnetic beads after the washing, and the materials were mixed by tapping. After that, 2 µL of M-MLV Reverse Transcriptase (Promega Corporation) was added to the mixture, and the whole was incubated at 42°C for 30 minutes while being rotated with a rotator.

Subsequently, the supernatant of the magnetic beads was discarded, and the residue was washed with 100 µL of a 1× binding buffer once. After that, the washed product was washed with 50 µL of an equilibration buffer (composition: 50 mM Tris-HCl (pH: 7.5), 150 mM NaCl, 5 mM imidazole, and 0.05% polyoxyethylene (20) sorbitan monolaurate) once. 99.5 Microliters of the equilibration buffer and 0.5 µL of RNase T1 (Thermo Fisher Scientific, Inc.) were added to the washed product, and the mixture was incubated at 37°C for 20 minutes while being rotated with a rotator, followed by the recovery of the supernatant. The solution thus recovered is an unpurified cDNA display library containing cDNA display molecules.

100 Microliters of HisPur Ni-NTA Magnetic Beads (Thermo Fisher Scientific, Inc.) were washed with 100 µL of the equilibration buffer once. The recovered cDNA display library was loaded into the washed product, and the mixture was shaken at room temperature for 30 minutes. After the shaking, the supernatant was discarded, and the residue was washed with 100 µL of the equilibration buffer twice. After that, 20 µL of an elution buffer (composition: 50 mM Tris-HCl (pH: 7.5), 150 mM NaCl, 250 mM imidazole, and 0.05% polyoxyethylene (20) sorbitan monolaurate) was added to the washed product, and the materials were mixed by tapping. The supernatant was recovered, and then 20 µL of the elution buffer was added thereto again, followed by mixing by tapping. The supernatant was similarly recovered, and its total volume was set to 40 µL.

Imidazole in the recovered solution was removed by dialysis. The dialysis was performed with Slide-A-Lyzer MINI (3.5 kDa MWCO) (Thermo Fisher Scientific, Inc.). The solution was stirred against 60 mL of a dialysis buffer (PBS) at 4°C for 60 minutes, and then the dialysis buffer was replaced with a new one, followed by further stirring at 4°C for 60 minutes. The solution recovered by the dialysis was adopted as a cDNA display library, and was used in subsequent screening.

### [Experimental Example 2: Screening of Peptide penetrating through each of Gastrointestinal Epithelial Cells]

### <Cell Culture>

ad-MED Vitrigel^{™} 2 Culture Insert (Kanto Chemical Co., Inc.) was set in a 24-well plate, and human iPS cell-derived intestinal epithelial cells "F-hiSIEC^{™} (FUJIFILM Corporation)" suspended in F-hiSIEC^{™} Seeding Medium (FUJIFILM Corporation) were seeded at 1.0×10⁵ cells/well. The cells were cultured under 37°C and 5% CO₂ in accordance with a manual manufactured by FUJIFILM Corporation, and a culture medium was replaced with a new one in accordance with the schedule of Culture Medium Replacement and Usage Example 2 described in the manual (F-hiSIEC^{™} Culture Medium (FUJIFILM Corporation) was used as a culture medium during a culture period). When the start date of the culture was defined as a first day, a first penetration assay (round 1) was performed on a 12th day, and a second penetration assay (round 2) was performed on a 14th day.

### <First Penetration Assay>

As peptide sequences for the variable region of the cDNA display library, linear peptides consisting of random 10-, 12-, or 15-mer amino acid sequences and cyclic peptides consisting of random 10-, 11-, or 12-mer sequences having cysteine residues at both termini were used. Three cDNA display libraries presenting linear peptides of different lengths, or three libraries presenting cyclic peptides of different lengths, were combined to prepare two mixed libraries of a linear-mixed type and a cyclic-mixed type), which were separately subjected in parallel to penetration assays. Each mixed library was suspended in PBS, and then diluted with fetal bovine serum (FBS)-free RPMI 1640 culture medium. After removing the medium from the culture inserts and wells, 150 µL of each diluted ibrary solution (corresponding to about 100 fmol to 300 fmol of cDNA display molecules) was added to separate culture inserts (apical side), and 600 µL of RPMI 1640 culture medium was added to each well (basolateral side) to initiate the penetration assay. The cells were incubated under 37°C and 5% CO₂ for 1 hour, and then the medium on the receptor (basolateral) side was entirely collected in 1.5-milliliter tubes. Each collected solution was stored at -80°C until nucleic acid quantitation and preparation of the next-round display library.

### <Purification and Amplification of Penetrated Sample>

About 500 µL of the sample recovered in the penetration assay was loaded into a centrifugal ultrafiltration device (Amicon Ultra 10 kDa MWCO, Millipore UFC5010), and was centrifuged at 4°C and 14,000 rpm for 10 minutes (TOMY TMP-24, rotation radius Rmax: 87 mm), followed by discarding the filtrate. The filter membrane was inverted onto a new tube and centrifuged at 4°C and 1,000 g for 2 minutes to collect the eluate (70 µL). DNA was purified using a DNA purification kit (QIAquick PCR Purification Kit QIAGEN 28104), and a 25 µL DNA sample was recovered. The DNA sample was subjected to PCR amplification in accordance with a common method to prepare the cDNA display library for the second round.

### <Second Penetration Assay, and Purification and Amplification of Penetrated Sample>

The second penetration assay was performed in the same manner as in the first penetration assay except that the cDNA display library for the second round was used, and the entirety of the culture medium on the receptor (basolateral) side was recovered in a 1.5-milliliter tube. A cDNA display library for the third round was produced from the recovered liquid in the same manner as that described above.

### <Third and Fourth Penetration Assays, and Purification and Amplification of Penetrated Samples>

For the third and fourth penetration assays, new human iPS cell-derived intestinal epithelial cells (F-hiSIEC^{™}) were prepared in the same manner as for the first and second penetration assays. Using the third-round cDNA display library, the penetration assay and the purification and amplification of the penetrated samples were performed as described above, thereby preparing the fourth-round cDNA display library.

Using the fourth-round cDNA display library, the penetration assay and the purification and amplification of the penetrated samples were likewise carried out as described above.

### <Next-generation Sequencing (NGS) Analysis>

Samples recovered in each penetration assay were used to prepare next-generation sequencing (NGS) libraries by appending adapter sequences via standard PCR, with reference to publicly available materials from Illumina, Inc. on the Tailed-PCR workflow. The libraries were analyzed on a MiniSeq^{™} (Illumina) instrument. The resulting paired-end FASTQ files (two FASTQ files) were analyzed using FLASH2, whereby the amino acid sequences of peptides capable of penetrating the plasma membrane were determined. Sequences with higher read counts in the fourth penetration assay are listed below.

**Table 2**

| Peptide No. | Amino acid sequence | SEQ ID NO |
|---|---|---|
| 1-1 | CKMWSPYGQETYC | 1 |
| 1-2 | CNRYRMIVDQSC | 2 |
| 1-3 | CSKNWYGWMFC | 3 |
| 1-4 | FQIYKFAFMV | 4 |
| 1-5 | GWKIPYLWHV | 5 |
| 1-6 | GYYKRMIKQQ | 6 |
| 1-7 | LFKAWLGVYH | 7 |
| 1-8 | MNYHKSNRHN | 8 |
| 1-9 | RVVSYLAEYH | 9 |
| 1-10 | VDMKSSNRLW | 10 |
| 1-11 | YFDDWLAPYH | 11 |

### [Experimental Example 3: Screening of Peptide capable of translocating into Lung Cancer A549 Cells]

A549 cells (Riken BioResource Research Center) were cultured in RPMI 1640 culture medium (ATCC modification, Thermo Fisher Scientific, Inc., product number: A10491-01)+10% FBS (containing penicillin-streptomycin, Thermo Fisher Scientific, Inc., product number: "15140-122", accounting for 1/100 of the volume of the mixture), and were seeded into a 24-well plate at 0.5×10⁵ cells/well. The cells were cultured under 37°C and 5% CO₂ for 72 hours, and then the culture medium in each well was removed. A cDNA display library equivalent to that of Experimental Example 2 was prepared (a total of six libraries presenting peptides with different number of amino acid residues: three linear and three cyclic). These were combined and diluted with RPMI 1640 culture medium that was free of FBS and antibiotics to a total volume of 310 µL, and 300 µL of the dilution (corresponding to about 100 fmol to 300 fmol of cDNA display molecules) was added to each well. After 1 h of uptake at 37°C and 5% CO₂, the applied library solution was recovered. 300 µL of fresh medium was then added, the culture was continued for 23 hours, and the cells were washed as described below.

The cells were washed with 300 µL of PBS three times. After that, the cells were washed with 300 µL of PBS+0.05% Tween 20 three times, and were washed with 300 µL of 10 mM hydrochloric acid+150 mM NaCl once. After that, 300 µL of 10 mM HEPES buffer (pH: 7.0) was added to the cells on ice, and the mixture was subjected to hypotonic treatment for 15 minutes. After confirming cell rupture by microscopic observation, the sample containing the intracellular contents was completely collected into a 1.5-mL tube. The supernatant was obtained by centrifugation at 1,000 × g for 3 min. Nucleic acids were purified and amplified from the supernatant in the same manner as in Experimental Example 2. Thus, a cDNA display library for the next round was prepared.

The above operations were repeated two additional times (three selection rounds in total), and the samples recovered as intracellular contents were subjected to next-generation sequencing (NGS) analysis. The resulting paired-end FASTQ files were analyzed using FLASH2, whereby the amino acid sequences of peptides that translocate into cells were determined. Sequences with higher read counts are listed below.

**Table 3**

| Peptide No. | Amino acid sequence | SEQ ID NO |
|---|---|---|
| 2-1 | CIRYYVARHVRRRC | 12 |
| 2-2 | CRMAPQFNHVC | 13 |
| 2-3 | LFKAWLGVYH | 7 |
| 2-4 | RVVSYLAEYH | 9 |
| 2-5 | RVVSYLAEYQ | 14 |
| 2-6 | TVTRGERYKH | 15 |
| 2-7 | VHSWVDMAKN | 16 |
| 2-8 | YFDDWLAPYH | 11 |
| 2-9 | YVDDWLAPYH | 17 |

### [Experimental Example 4: Screening of Peptide capable of translocating into Pancreatic Cancer Panc-1 Cells]

Panc-1 cells (Riken BioResource Research Center) were cultured in the same RPMI 1640 culture medium+10% FBS (containing penicillin-streptomycin) as that of Experimental Example 3, and were seeded into a 24-well plate at 0.5×10⁵ cells/well. Using the method described in Experimental Example 3, two selection rounds were conducted in total, and the amino acid sequences of peptides that translocate into cells were determined. Sequences with higher read counts in NGS analysis are listed below.

**Table 4**

| Peptide No. | Amino acid sequence | SEQ ID NO |
|---|---|---|
| 3-1 | CIRYYVARHVRRRC | 12 |
| 3-2 | LFKAWLGVYH | 7 |
| 3-3 | QYNMVRQDLI | 18 |
| 3-4 | RVVSYLAEYH | 9 |
| 3-5 | VRVKTYWENH | 19 |

When the same experiment was performed by using mRNA display library instead of the cDNA display libraries in Experimental Example 3, with regard to the residual ratio of a nucleic acid 1 hour after its cell contact, the residual ratios of linear peptide display molecules and cyclic peptide display molecules in the cDNA display libraries were each more than 90%, but the residual ratio of linear peptide display molecules in the mRNA display library was 0.47%. In addition, when the same experiment was performed by using the mRNA display library instead of the cDNA display libraries in Experimental Example 4, with regard to the residual ratio of a nucleic acid 1 hour after its cell contact, the residual ratios of linear peptide display molecules and cyclic peptide display molecules in the cDNA display libraries were 23% and 51%, respectively, but the residual ratio of the linear peptide display molecules in the mRNA display library was 0.76%. It is found from the foregoing that according to cDNA display method, high-stability screening can be performed even in a system using living cells.

### [Experimental Example 5: Screening of Peptides that translocate into neutrophils]

HL-60 cells (Riken BioResource Research Center) were cultured in RPMI 1640 medium supplemented with 10% FBS (containing penicillin-streptomycin), and were seeded into a 24-well plate at 1.0×10⁵ cells/well. Dimethyl sulfoxide (DMSO, FUJIFILM Wako Pure Chemical Corporation, 041-29351) was added to a final concentration of 1.3%, and the cells were incubated at 37°C and 5% CO₂ for 72 hours to differentiate the HL-60 cells into neutrophils.

The culture medium of each well was removed, and a cDNA display library equivalent to that used in Experimental Example 3 was diluted with RPMI 1640 medium that was free of FBS and antibiotics to a total volume of 310 µL, and 300 µL of the dilution was added to each well. The cDNA display molecules were taken up under 37°C and 5% CO₂ for 1 hour, and then the added libraries were recovered. After that, 300 µL of PBS was added to each well, and the cells were collected by pipetting, followed by the washing of the cells by centrifugation at 200 g for 3 minutes. Further, the cells were washed with 300 µL of PBS+0.05% Tween 20, and were similarly collected by centrifugation. Subsequently, on ice, 300 µL of 10 mM HEPES buffer (pH 7.0) was added, and the cells were subjected to hypotonic treatment for 15 minutes. After confirming cell rupture by microscopic observation, the sample containing the intracellular contents was completely collected into a 1.5-mL tube. The supernatant was obtained by centrifugation at 1,000 × g for 3 min. The supernatant was purified and amplified in the same manner as in Experimental Example 2 to prepare the cDNA display library for the next round.

The above operations were repeated four additional times (five selection rounds in total), and the samples recovered as intracellular contents were subjected to NGS analysis. The resulting paired-end FASTQ files were analyzed using FLASH2, whereby the amino acid sequences of peptides that translocate into cells were determined. Sequences with higher read counts are listed below.

**Table 5**

| Peptide No. | Amino acid sequence | SEQ ID NO |
|---|---|---|
| 4-1 | FLPMFSRLGLQI | 20 |
| 4-2 | FQIYKFAFMV | 4 |
| 4-3 | FSVRLPMFSFRL | 21 |
| 4-4 | IQIKVMAWLT | 22 |
| 4-5 | KWPVINVEVK | 23 |
| 4-6 | LFARLLDMFF | 24 |
| 4-7 | LYARILNWIF | 25 |
| 4-8 | LYHTAIKTFT | 26 |
| 4-9 | QALLKLYFSF | 27 |
| 4-10 | RLIFRHVIDMIF | 28 |
| 4-11 | RVVSYLAEYH | 9 |
| 4-12 | VHVHVMINFF | 29 |
| 4-13 | WYMVHLRSEW | 30 |

### [Experimental Example 6: Screening of Peptides that translocate into macrophages]

THP-1 cells (Riken BioResource Research Center) were cultured in RPMI 1640 medium supplemented with 10% FBS (containing penicillin-streptomycin), and were seeded into a 24-well plate at 1.0×10⁵ cells/well. A DMSO stock solution (10 µg/mL) of phorbol 12-myristate 13-acetate (PMA, Funakoshi Co., Ltd., AG-CN2-0010-M001) was added at 1/1000 (v/v) to give a final concentration of 10 ng/mL PMA, and the cells were incubated at 37°C and 5% CO₂ for 24 hours. The medium was removed, and 300 µL of fresh RPMI 1640 medium+10% FBS was added to each well, followed by incubation for 24 hours. The respective stock solutions of IFN γ (Cosmo Bio Company, Limited (Proteintech Group, Inc.), HZ-1301) and LPS (Cosmo Bio Company, Limited (Santa Cruz Biotechnology), SC-3535) were added to the wells so that their final concentrations were 20 ng/mL and 250 ng/mL, respectively, followed by further incubation at 37°C and 5% CO₂ for 24 hours. Thus, the THP-1 cells were differentiated into macrophages.

After removing the medium from each well, a cDNA display library equivalent to that used in Experimental Example 3 was diluted with RPMI 1640 medium that was free of FBS and antibiotics to a total volume of 310 µL, and 300 µL of the dilution was added to each well. The cDNA display molecules were taken up at 37°C and 5% CO₂ for 1 hour, and then the cells were washed as described below.

The cells were washed with 300 µL of PBS three times. After that, the cells were washed with 300 µL of PBS+0.05% Tween 20 three times, and were washed with 300 µL of 10 mM hydrochloric acid+150 mM NaCl once. After that, 300 µL of 10 mM HEPES buffer (pH 7.0) was added on ice, and the cells were subjected to hypotonic treatment for 15 minutes. After confirming cell rupture by microscopic observation, the sample containing the intracellular contents was completely collected into a 1.5-mL tube. The supernatant was obtained by centrifugation at 1,000 × g for 3 min. The supernatant was purified and amplified in the same manner as in of Experimental Example 2 to prepare the cDNA display library for the next round.

The above operations were repeated three additional times (four selection rounds in total), and the samples recovered as intracellular contents were subjected to NGS analysis. The resulting paired-end FASTQ files were analyzed using FLASH2, whereby the amino acid sequences of peptides that translocate into cells were determined. Sequences with higher read counts are listed below.

**Table 6**

| Peptide No. | Amino acid sequence | SEQ ID NO |
|---|---|---|
| 5-1 | SHYSLIVFSR | 31 |
| 5-2 | CH MVNTYKWTFFIC | 32 |
| 5-3 | CTPWKNRNVLLWYC | 33 |
| 5-4 | HFHYYMHSVY | 34 |
| 5-5 | KILIFHYIFV | 35 |
| 5-6 | RWLIFHYMLL | 36 |
| 5-7 | RWPLWMWFVW | 37 |
| 5-8 | RYYAHWLHYM | 38 |
| 5-9 | YYLLPLHVKH | 39 |

### [Experimental Example 7: Screening of Peptide penetrating through BBB Model]

### <Cell Culture>

A monkey BBB kit (6 inserts/24-well; PharmaCo-Cell Company Ltd., PCC-MBT-24Q) was cultured according to the manufacturer's manual. DMEM/F-12 medium (Gibco, 11330-032)+0.5% BSA (FUJIFILM Wako Pure Chemical Corporation, 017-22231)+500 nM hydrocortisone was used as the assay buffer. Defining the day of culture initiation as Day 1, the first penetration assay (Round 1) was performed on Day 5, and the second (Round 2) on Day 7.

### <First Penetration Assay>

As peptide sequences for the variable region of the cDNA display library, linear peptides consisting of random 8-, 10-, or 12-mer amino acid sequences and cyclic peptides consisting of random 9-, 10-, or 11-mer sequences having cysteine residues at both termini were used. Three libraries presenting linear peptides with different number of amino acid residues or three libraries presenting cyclic peptides with different number of amino acid residues were combined to prepare two mixed cDNA display libraries of a linear mixed type and a cyclic mixed type), which were separately subjected in parallel to the penetration assays. Each mixed cDNA display library was suspended in PBS to obtain two mixed-library solutions.

On the 5th day from the start of the culture, the inserts culturing the BBB were each transferred to a well for washing once, and the culture medium in the insert was carefully removed with an aspirator. After that, the insert was transferred to a well for an assay having added thereto 900 µL of the assay buffer in advance. 20 Microliters of each of the cDNA display library solutions was mixed and diluted with 200 µL of the assay buffer by adding the buffer thereto, and 200 µL each of the diluted solutions was added to a separate insert to initiate the penetration assay. The BBBs were incubated at 37°C and 5% CO₂ for 2 hours, and then each receptor solution was collected.

### <Purification and Amplification of Penetrated Sample>

The collected solutions were purified and amplified in the same manner as in Experimental Example 2, and used to prepare the cDNA display library for the next round.

### <Second Penetration Assay, and Purification and Amplification of Penetrated Sample>

On the 7th day from the start of the culture, the second penetration assay was conducted in the same manner as the first penetration assay except that the second-round cDNA display library was used. Each receptor solution was collected and, after purification and amplification as in Experimental Example 2, was used to prepare the third-round cDNA display library.

### <Third and Fourth Penetration Assays, and Purification and Amplification of Penetrated Samples>

A new monkey-type BBB kit (6 inserts/24-wells; PharmaCo-Cell Company Ltd.) was prepared for each of third and fourth penetration assays in the same manner as for the first and second assays. Using the third-round cDNA display library, the penetration assay and the purification and amplification of the penetrated samples were performed as described above, thereby preparing the fourth-round cDNA display library.

Using the fourth-round cDNA display library, the penetration assay and the purification and amplification of the penetrated samples were likewise carried out as described above.

The degree of the convergence of the amino acid sequence of the sample recovered in each of the penetration assays is recognized by subjecting the sample to next-generation sequencing (NGS) analysis, and the above-mentioned penetration assay is repeatedly performed until the sequence sufficiently converges. When sufficient convergence is confirmed, the sequences exhibiting higher read counts at that time are identified as the amino acid sequences of the cell-penetrating peptides.

### [Experimental Example 8: Screening of Peptide penetrating through Skin Model]

A three-dimensional skin model EpiDerm Full Thickness 400 (manufactured by Kurabo Industries Ltd., #EFT-400) similar to human skin was cultured in accordance with the manual of its manufacturer with a culture medium included in the kit. Thus, a skin model was built on the donor side (inner side) of a collagen-coated culture cup having a size corresponding to a 24-well plate. 100 Microliters of a peptide library was added to the donor side of the culture cup to start screening. The mixture was cultured for 24 hours after the addition, and then 2.5 mL of a culture solution on the receptor side of the cup was recovered. The culture solution was set in an ultrafiltration centrifugal filter (Amicon Ultra 10 kDa MWCO, Millipore UFC5010), and was centrifuged at 4°C and 14,000 rpm for 10 minutes (TOMY TMP-24, rotation radius Rmax: 87 mm), followed by its concentration to about 100 µL. DNA purification was performed with a DNA purification kit (QIAquick PCR Purification Kit QIAGEN 28104), and 25 µL of a DNA sample was recovered.

The DNA sample was subjected to PCR amplification according to standard methods to prepare a cDNA display library for the second round. Rounds of penetration assays were similarly repeated, and a total of 4 rounds were performed at most.

The samples recovered in each of the penetration assays were subjected to next-generation sequencing (NGS) analysis, and the resulting paired-end FASTQ files were analyzed using FLASH2, whereby the amino acid sequences of peptides that penetrated the skin model were determined. "RWNCVSCAFL" (SEQ ID NO: 40) was identified as a sequence with high read counts.

### [Experimental Example 9: Assay of Peptide for Skin Penetrating Activity]

A three-dimensional skin model EpiDerm Full Thickness 400 was prepared in accordance with the manual of its manufacturer in the same manner as in Experimental Example 8. Meanwhile, the N-terminal side of the peptide represented by SEQ ID NO: 40 was modified with fluorescein amidite (FAM) (synthesized by Eurofins Genomics K.K., theoretical molecular weight: 1,918.1) through a PEG8 linker. As a control, the known cell-penetrating peptide TAT (the sequence obtained by removing C at the N-terminal from the amino acid sequence shown in SEQ ID NO: 51) was used, and the N-terminal side thereof was modified with FAM (similarly synthesized by Eurofins Genomics K.K., theoretical molecular weight: 1,979.2).

100 Microliters of a 1% DMSO-containing PBS solution of each of the FAM-modified peptides was added to a central portion on the donor side of a cup in which the skin model was built to initiate the penetrability assay (n=3). After incubation at 37 °C under 5% CO₂ for 24 h, the medium outside the cup was collected, and the concentration of peptide that penetrated the skin model was quantified fluorometrically using a microplate reader (TECAN Spark; excitation wavelength: 494 nm, emission wavelength: 521 nm).

As a result, the ratio of penetrated amount of the FAM-labeled TAT peptide outside the cup to its added amount, that is, the penetrability thereof was 21.4±3.3% (mean ± SD, n = 3). The penetrability of the FAM-labeled SEQ ID NO: 40 peptide was 49.8±10.0% (mean ± SD, n = 3), and hence the penetrability was significantly higher than that of the TAT sequence (p<0.01).

### [Experimental Example 10: Preparation of eGFP-peptide Fusion Protein]

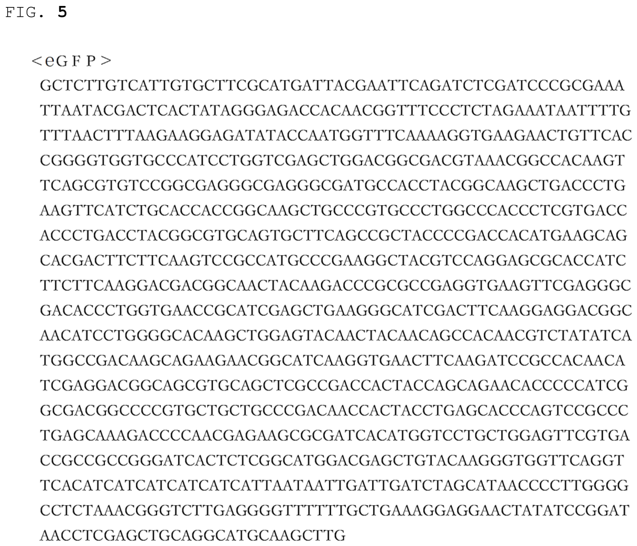

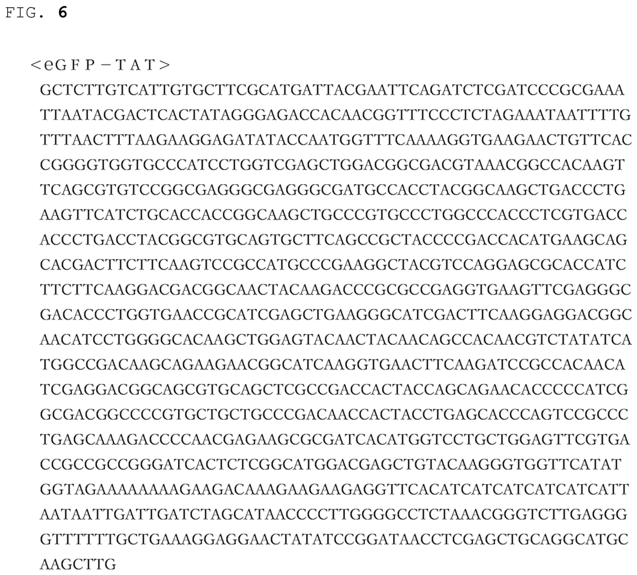

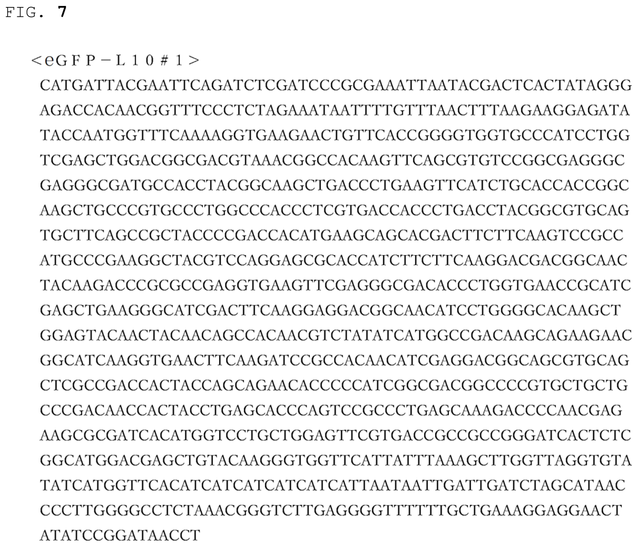

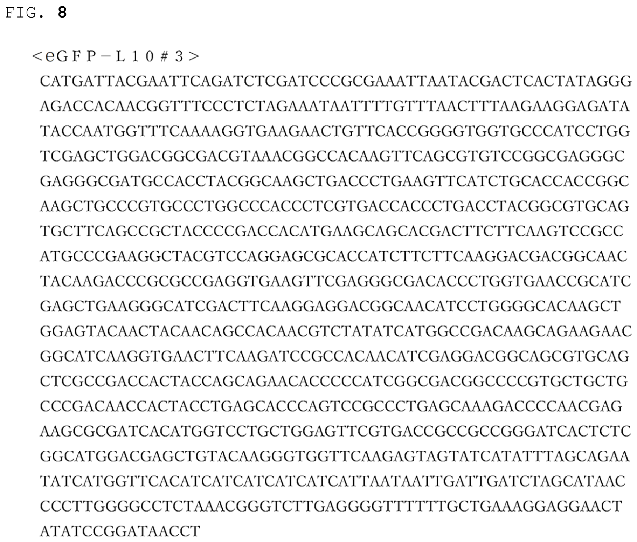

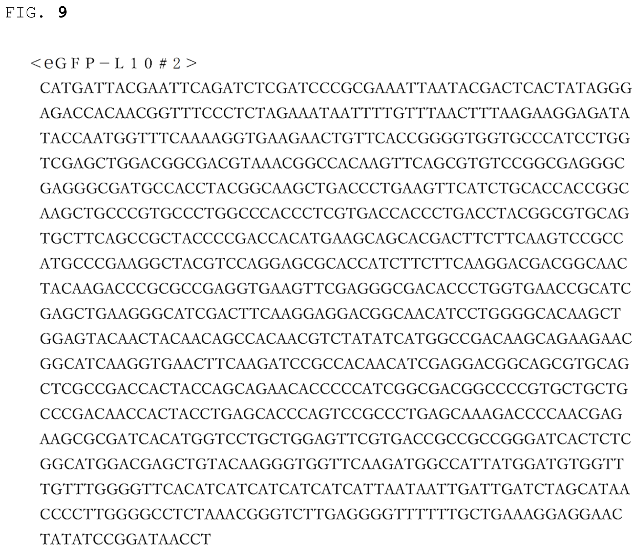

Template DNA required for expression of eGFP or eGFP-peptide fusion proteins, including DNA required for protein expression in a cell-free system, was synthesized by Eurofins Genomics upon request. The synthesized template DNAs are as described below, and their DNA sequences are shown in FIG. **5** to FIG. **9****.**

**Table 7**

| Protein name | SEQ ID NO | |
|---|---|---|
| eGFP | 41 | |
| eGFP-TAT | 42 | DNA encoding eGFP-peptide fusion protein obtained by adding TAT peptide formed of amino acid sequence YGRKKRRQRRR to lysine residue at 239th position of eGFP encoded by DNA as set forth in SEQ ID NO: 41 through GGS linker |
| eGFP-L10#1 | 43 | DNA encoding eGFP-peptide fusion protein obtained by adding peptide including amino acid sequence as set forth in SEQ ID NO: 7 to lysine residue at 239th position of eGFP encoded by DNA as set forth in SEQ ID NO: 41 through GGS linker |
| eGFP-L10#3 | 44 | DNA encoding eGFP-peptide fusion protein obtained by adding peptide including amino acid sequence as set forth in SEQ ID NO: 9 to lysine residue at 239th position of eGFP encoded by DNA as set forth in SEQ ID NO: 41 through GGS linker |
| eGFP-L10#2 | 45 | DNA encoding eGFP-peptide fusion protein obtained by adding peptide including amino acid sequence as set forth in SEQ ID NO: 37 to lysine residue at 239th position of eGFP encoded by DNA as set forth in SEQ ID NO: 41 |

| | | |
|---|---|---|
| TAT is a known membrane-permeable peptide. | | |

About 5 µg of each DNA fragment was obtained. Using a portion (0.25-1 µg), eGFP and eGFP-peptide fusion proteins were prepared and purified according to the following procedure using a "MUSAIBO KUN^{™} N100" (Taiyo Nippon Sanso Corporation, SI division).

Each of the template DNAs was dissolved in a 10 mM Tris-HCl buffer (pH: 7.5) (about 100 µg/mL), and, in accordance with the manual of the manufacturer of the kit, the solution was further diluted with purified water to a predetermined concentration, followed by its mixing with a reaction liquid premix included in the kit. Thus, the total volume was finally set to 100 µL (reaction liquid). Subsequently, 1 mL of a dialysis buffer included in the kit was loaded into the outside of a dialysis unit, and the reaction liquid was loaded into the dialysis unit. After the unit had been shaken at 30°C for 16 h (amplitude: 25 mm, 80 rpm, BIOSHAKER BR-53FP, Taitec Corporation), the reaction liquid was recovered in a new tube, and was left to stand still on ice for 5 minutes. Thus, the reaction was stopped. After that, the volume of the resultant was adjusted to about 1,000 µL with PBS+0.05% Tween 20, and the resultant was purified with HisPur^{™} Ni-NTA Magnetic Beads (Thermo Fisher Scientific^{™}, Inc., product number: 88831). The beads were equilibrated in advance by the following procedure.

100 Microliters of a slurry of the magnetic beads was transferred to a new 1.5-milliliter tube, and 400 µL (fourfold amount) of an equilibration buffer (PBS+0.05% Tween 20) was added thereto, followed by suspension with Vortex (weak) for 10 seconds. The beads were collected with a magnetic stand, and the supernatant was removed. 1,000 Microliters (tenfold amount) of the equilibration buffer was further added to the beads, and the beads were suspended therein with Vortex (weak) for 10 seconds. The supernatant was similarly removed, and the beads were recovered and equilibrated.

The reaction liquid was added to the equilibrated beads, and the beads were suspended therein. The materials were mixed by rotation at 4°C for 1 h, and then the beads were collected with a magnetic stand. The beads were washed with about 1 mL of PBS+0.05% Tween 20+5 mM imidazole three times. Subsequently, the washed product was eluted with the following buffers, and a yellowish green fraction was recovered.

That is, the elution was performed with 20 µL of PBS+250 mM imidazole twice, with 20 µL of PBS+250 mM imidazole+1 mM EDTA twice, and with 20 µL of PBS+250 mM imidazole+10 mM EDTA twice.

The eGFP and the eGFP-peptide fusion protein thus recovered were each used in the following cell assay after its medium had been replaced with 20 mM HEPES (pH: 7.0)+150 mM NaCl by using an ultrafiltration membrane (Amicon Ultra 10 kDa 0.5 mL Millipore UFC501024). In addition, the concentration of each of the eGFP and the eGFP-peptide fusion protein was calculated on the basis of the absorption coefficient thereof at 280 nm calculated from its amino acid sequence.

### [Experimental Example 11: Intracellular translocation assay of eGFP-peptide Fusion Proteins in A549 Lung Cancer Cells]

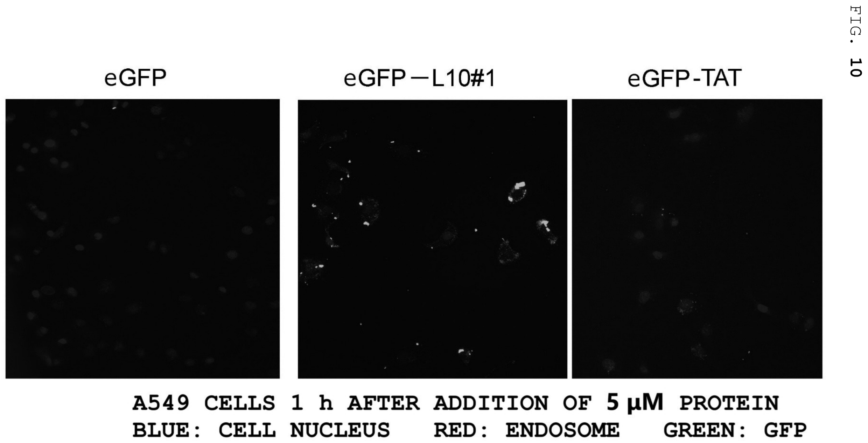

A549 cells suspended in RPMI 1640 medium+10% FBS (containing penicillin-streptomycin) were seeded in a 96-well plate at 5,000 cells/well and incubated at 37°C and 5% CO₂ for 24 hours. After removing the medium, the cells were washed with 200 µL of RPMI 1640 medium that was free of FBS and antibiotics once. Protein solution (eGFP, eGFP-TAT, or eGFP-L10#1) was diluted in the same RPMI 1640 medium and added to each of the wells (final concentration 5 µM, 30 µL/well). After incubation at 37°C and 5% CO₂ for 30 minutes, LYSO-ID^{™} Red detection kit reagent (Cosmo Bio Company, Limited, product number: ENZ-51005-0100) was diluted 500-fold in RPMI 1640 medium, and then 10 µL of the diluted reagent was added to each well. Following a further 15 min of incubation, 3 µL of NucBlue^{™} reagent (Thermo Fisher Scientific, Inc.) was added to each well. After an additional 15 min incubation, the plate was cooled on ice and washed three times with 200 µL of PBS containing 0.5 mg/mL heparin sulfate cooled to 4°C. The cells were further washed twice with 100 µL of Live Cell Imaging Solution (Thermo Fisher Scientific, Inc.) cooled to 4°C, after which 50 µL of the same solution was added and the cells were examined by fluorescence microscopy. Imaging results acquired using an all-in-one fluorescence microscope "BZ-X810" (Keyence Corporation) are shown in FIG. 10.

As shown in FIG. 10, in the sample using eGFP-L10#1, which is a fusion protein of eGFP with a peptide including the amino acid sequence of SEQ ID NO: 7, eGFP-derived green fluorescence was observed so as to surround nucleus-derived blue fluorescence, indicating that the eGFP-L10#1 efficiently translocated into the cytosol. Herein, the molecular weight of the eGFP is 27 kDa, and the molecular weight of the peptide appended to eGFP is 1.2 kDa. By contrast, in the samples using eGFP alone or eGFP-TAT which is a fusion protein of eGFP with the known cell-penetrating peptide TAT, only very faint perinuclear eGFP fluorescence was observed; accordingly, little cytosolic translocation of eGFP or the eGFP fusion protein was confirmed.

### [Experimental Example 12: Intracellular translocation assay of eGFP-peptide Fusion Proteins in THP-1 Macrophages]

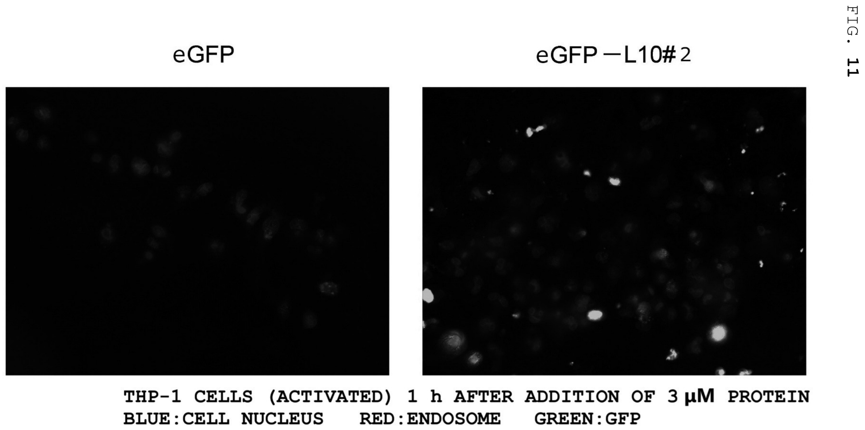

THP-1 macrophage cells, which had been seeded in a 96-well plate at 5,000 cells/well, and had already been activated with PMA, IFN γ, and LPS, were used. After removing the medium, the cells were washed once with 200 µL of RPMI 1640 culture medium that was free of FBS and antibiotics, and protein solution (an eGFP or eGFP-L10#2) diluted in the same medium was added to each of the wells (final concentration 3 µM, 30 µL/well). The cells were examined 1 h after addition following the same procedure as in Experimental Example 11. The results are shown in FIG. 11.

As shown in FIG. **11****,** in the sample using eGFP-L10#2, which is a fusion protein of eGFP with a peptide including the amino acid sequence of SEQ ID NO: 37, eGFP-derived green fluorescence was observed together with nucleus-derived blue fluorescence, indicating that eGFP-L10#2 translocated into macrophages. Herein, the molecular weight of eGFP is 27 kDa, and the molecular weight of the peptide appended to eGFP is 1.5 kDa. By contrast, in the sample using eGFP alone, no eGFP fluorescence was observed; accordingly, intracellular translocation of eGFP into macrophages was not confirmed.

### [Experimental Example 13: Intracellular translocation assay of Alexa 647-labeled IgG in Macrophages]

### <Labeling of IgG with Alexa 647>

Alexa Fluor 647 NHS Ester (Thermo Fisher Scientific, Inc., #A20006) was dissolved in DMSO so that its concentration was 10 mM. A hIgG (normal human IgG, whole molecule, purified product) (Takara Bio Inc., #143-09501) was dissolved in PBS so that its concentration was 10 mg/mL. 1 Milligram of the hIgG was added to a 1.5-milliliter tube so that its final concentration was 5 mg/mL, and the Alexa Fluor 647 NHS Ester was added thereto so that its molar amount was 10 times as large as that of the hIgG (total volume: 200 µL), followed by stirring by inversion at 25°C for 30 minutes. The reaction liquid was applied to a Zeba spin desalting column (7K MWCO) (Thermo Fisher Scientific, Inc., #89882) equilibrated with PBS, and an unreacted reagent was removed. Thus, an Alexa 647-labeled IgG was recovered. The absorbance values of the Alexa 647-labeled IgG at 280 nm and 650 nm were measured as described in the manual of the manufacturer of the ester. As a result, it was calculated that one molecule of the recovered IgG was labeled with about three molecules of the Alexa 647.

### <Addition of Peptide to Alexa 647-labeled IgG>

A 10 mM DMSO solution of Maleimide-PEG2-NHS Ester (Tokyo Chemical Industry Co., Ltd., product code: M3339) serving as a cross-linking agent including a hydrophilic linker or a 10 mM PBS solution of a Sulfo-SMCC cross-linking agent was added to about 1 mg of the Alexa 647-labeled IgG (PBS solution) so that its molar amount was 20 times as large as that of the Alexa 647-labeled IgG, followed by stirring by inversion at 25°C for 1 hour. The reaction liquid was applied to a Zeba spin desalting column (7K MWCO) equilibrated with PBS, and an unreacted cross-linking agent was removed. Thus, an IgG fraction was recovered. The peptides (synthesized by Eurofins Genomics K.K., the amino acid sequences of the peptides that are each conjugated with the IgG are shown in the following table) obtained by the above-mentioned screening, the peptides each including an amino acid sequence and each having C at its N-terminal, were each dissolved in DMSO so that a 10 mM DMSO solution was obtained. The DMSO solution of the peptide whose molar amount was 5 times as large as that of the IgG was added to the recovered IgG fraction, and EDTA was further added to the mixture so that its final concentration was 1 mM, followed by stirring by inversion at 25°C for 1 hour. After that, a fivefold molar amount of cysteine was added to the mixture, and the whole was stirred by inversion at 25°C for 30 minutes. The reaction liquid was applied to a Zeba spin desalting column (7K MWCO) equilibrated with PBS, and the peptide and cysteine that were unreacted, and EDTA were removed. Thus, an IgG-peptide conjugate was recovered. For example, when the peptide was T001, it was calculated from the measured absorbance values of the conjugate at 280 nm and 650 nm, and the measured value of a tryptophan-derived fluorescence intensity (excitation wavelength: 280 nm, emission wavelength: 350 nm) that three to four molecules of the peptide were added per one molecule of the IgG.

**Table 8**

| | Amino acid sequence (all C-terminals are amidated) | SEQ ID NO | Cross-linking agent |
|---|---|---|---|
| T001 | CRWPLWMWFVW | 46 | Maleimide-PEG2-NHS Ester |
| T005 | CGKEGGSRWLIFHYMLLGG | 47 | Maleimide-PEG2-NHS Ester |
| T006 | CRYYAHWLHYM | 48 | Sulfo-SMCC |
| R001 | CLFKAWLGVYH | 49 | Maleimide-PEG2-NHS Ester |
| R005 | CGGSRVVSYLAEYH | 50 | Sulfo-SMCC |
| TAT | CYGRKKRRQRRR | 51 | Maleimide-PEG2-NHS Ester |

| | | | |
|---|---|---|---|
| In the table, an underlined portion is the amino acid sequence of a membrane-permeable peptide identified by screening. | | | |

### <Intracellular Delivery of IgG to THP-1 Macrophages by Peptide Conjugation>

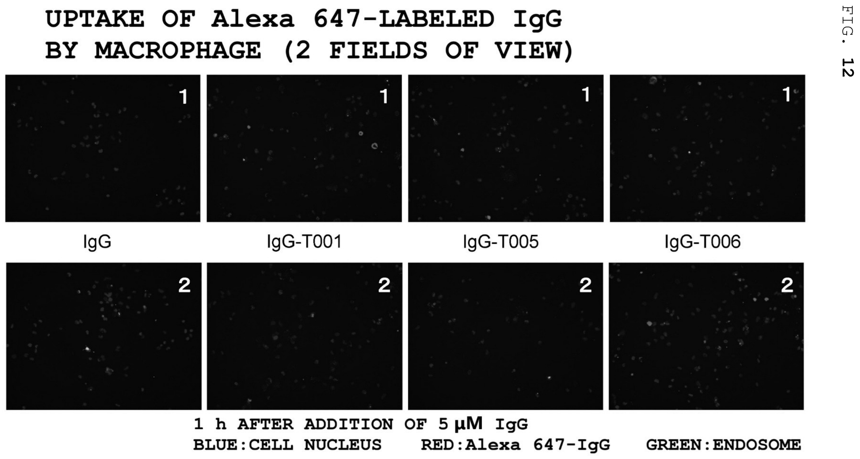

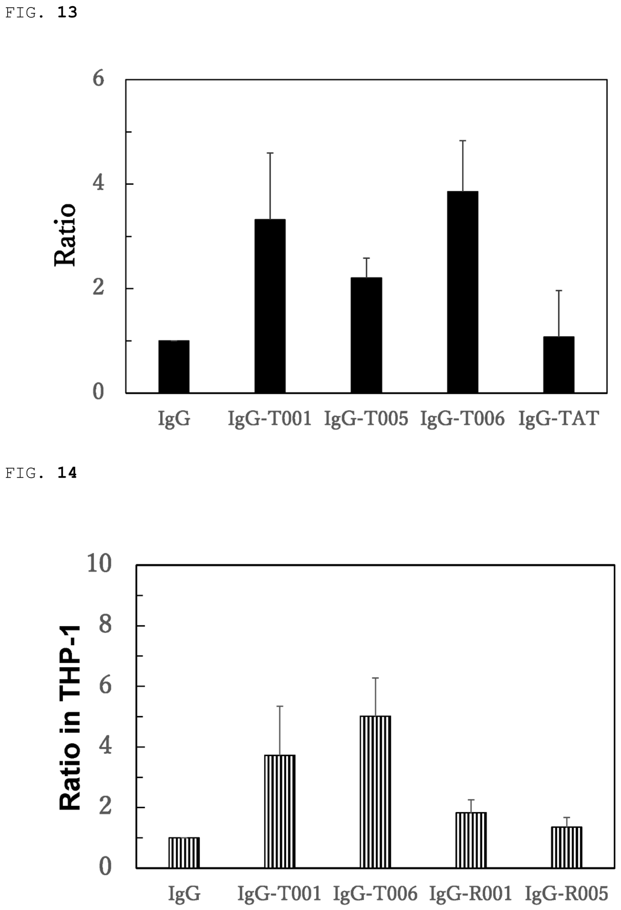

The assay was conducted using THP-1 macrophages, which had been seeded in a 96-well plate at 20,000 cells/well, and had already been activated with PMA, IFN γ, and LPS. After removing the medium from each well, the cells were washed once with 200 µL of RPMI 1640 medium that was free of FBS and antibiotics, and protein solution (IgG or IgG-peptide conjugate) diluted in the same RPMI 1640 medium was added to each of the wells (final concentration 5 µM, 30 µL/well). The cells were examined 1 h after addition following the procedure of Experimental Example 11. The results are shown in FIG. **12****.** Images acquired with the all-in-one fluorescence microscope BZ-X810 (Keyence Corporation) were analyzed using the accompanying software BZ-X800 Analyzer. The uptake ratio at an uptake time of 1 h was compared based on the ratio of integrated red fluorescence intensity to integrated blue fluorescence intensity. The results are shown in FIG. **13** (in the figure, bar heights indicate the average of "n" values (n=3) and error bars indicate the standard deviation).

As shown in FIG. **12** and FIG. **13****,** the bonding of peptides including the amino acid sequences obtained in the macrophage-cell screening (T001, T005, and T006) increased the intracellular delivery amount of IgG. By contrast, no increase in cellular translocation was observed for IgG bonded with TAT.

### [Experimental Example 14: Cell-Specificity Evaluation of Plasma membrane-Penetrating Activity 1]

The cells 1 hour after the addition of the protein solution were observed and photographed with an all-in-one fluorescence microscope BZ-X810 (Keyence Corporation) in the same manner as in Experimental Example 13 except that the protein solution was added to each of the wells so that the final concentration of the IgG or the IgG-peptide conjugate was 1 µM, followed by image analysis with software BZ-X800 Analyzer included therein. The uptake ratios of the proteins at an uptake time of 1 hour, which were each determined from the ratio of the brightness (integrated) of a red color to the brightness (integrated) of a blue color, were compared to each other. The results are shown in FIG. **14** (in the figure, the height of a bar represents the average of "n" values (n=2 to 3) and an error bar represents its standard deviation).

As shown in FIG. **14****,** IgG bonded with peptides including the amino acid sequences obtained in the macrophage-cell screening (T001, T006) exhibited a greater increase in the intracellularly delivered amount into macrophages than IgG bonded with peptides including the sequences obtained in the A549-cell screening (R001, R005).

### [Experimental Example 15: Cell-Specificity Evaluation of Plasma membrane-Penetrating Activity 2]

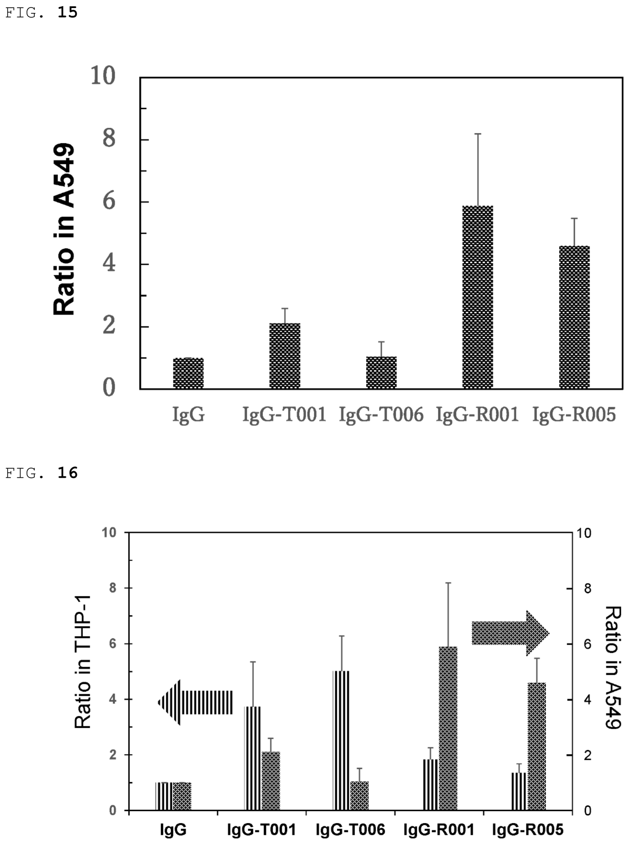

Cells 1 hour after the addition of the protein solution were observed and photographed with an all-in-one fluorescence microscope BZ-X810 (Keyence Corporation) in the same manner as in Experimental Example 14 except that A549 cells cultured in the same manner as in Experimental Example 11 were used, followed by image analysis with software BZ-X800 Analyzer included therein. The uptake ratios of the proteins at an uptake time of 1 hour, which were each determined from the ratio of the brightness (integrated) of a red color to the brightness (integrated) of a blue color, were compared to each other. The results are shown in FIG. **15** (in the figure, the height of a bar represents the average of "n" values (n=2 to 3) and an error bar represents its standard deviation).

As shown in FIG. **15****,** IgG bonded with peptides including the amino acid sequences obtained in the A549-cell screening (R001, R005) exhibited a greater increase in the intracellularly delivered amount into A549 cells than IgG bonded with peptides including the sequences obtained in the macrophage-cell screening (T001, T006).

The results of Experimental Examples 14 and 15 are collectively shown in FIG. **16** (in the figure, the height of a bar represents the average of "n" values (n=2 to 3) and an error bar represents its standard deviation). As shown in FIG. **16****,** these results indicate that peptides R001 and R005, which include the amino acid sequences obtained in the A549-cell screening, exhibit cell-type-selective plasma membrane-penetrating activity in A549 cells, whereas peptides T001 and T006, which include the amino acid sequences obtained in the macrophage-cell screening, exhibit cell-type-selective plasma membrane-penetrating activity in macrophages.

### [Experimental Example 17: Preparation of siRNA-encapsulated Lipid Nanoparticles (LNPs) and Modification thereof with Peptide]

siRNA-encapsulated LNPs were prepared with Lipid Nanoparticle (LNP-102) Exploration Kit (Cayman Chemical) in accordance with its manual.

However, 1,2-distearoyl-rac-glycero-3-(2'-maleimidoethyl)polyoxyethylene (DSG-PEG(2000)-maleimide) (SUNBRIGHT GS-020MA, Nippon Oil & Fats Co., Ltd.) was added as a lipid having a maleimide group for peptide modification.

As the siRNA, an anti-luciferase siRNA (siRNA(LUC)) having the sequence set forth below was custom-synthesized by Ajinomoto Bio-Pharma Services (Gene Design Inc.).
·siRNA(LUC)
   Sense strand: 5'-CUUACGCUGAGUACUUCGAdTdT (SEQ ID NO: 52)
   Antisense strand: 5'-UCGAAGUACUCAGCGUAAGdTdT (SEQ ID NO: 53)

1,2-Distearoyl-sn-glycero-3-phosphatidylcholine (DSPC), cholesterol, and DMG-PEG(2000) included in the kit were dissolved in ethanol. DSG-PEG(2000)-maleimide was dissolved in DMF.

Separetely, siRNA(LUC) was dissolved in autoclaved 50 mM sodium acetate buffer (pH 5.0).

Based on the lipid molar composition recommended in the manufacturer's manual (ionizable lipid SM-102 : neutral phospholipid DSPC : cholesterol : PEGylated lipid DMG-PEG(2000) = 50 : 10 : 38.5 : 1.5), a composition was employed in which the proportion of DMG-PEG(2000) was reduced by 0.5 and, instead, DSG-PEG(2000)-maleimide was added at 0.5. A 100 mg/mL stock solution was used as SM-102, a 25 mg/mL stock solution was used as DSPC, a 5 mg/mL stock solution was used as cholesterol, a 1 mg/mL stock solution was used as DMG-PEG (2000), and a 5 mg/mL stock solution was used as DSG-PEG(2000)-maleimide.

To achieve the above molar composition and the siRNA content recommended in the manual, stock solutions of each lipid were added to the siRNA solution in 50 mM sodium acetate (pH 5.0) and rapidly mixed by pipetting for 15 seconds.

The prepared sample (about 60 µL) was loaded into a microdialysis cartridge Xpress Micro Dialyzer MD100 having a molecular weight cut-off of 140 kDa (Scienova GmbH) (Funakoshi Co., Ltd. (SCI), code: 40931), the cartridge being set in a 2-milliliter tube. The sample was dialyzed against 1.2 mL of PBS (20 minutes, three times), and the recovered sample was used as maleimide group-bearing LNP.

Subsequently, a 10 mM DMSO solution of Cys-appended cell-penetrating peptide 1 (CTVTRGERYKH (SEQ ID NO: 54)) having cysteine at its N-terminal; 5 molar equivalents relative to maleimide groups; was added, and the mixture was incubated for 60 minutes at room temperature. The Cys-appended cell-penetrating peptide 1 has the amino acid sequence obtained by adding cysteine to the N-terminal of the peptide No. 2-6 (SEQ ID NO: 15).

After that, a fivefold molar amount of cysteine (10 mM PBS solution) was further added, and the mixture was incubated for 15 minutes at room temperature. After dialysis as described above, the recovered LNP was designated the peptide-modified LNP (abbreviation: A001), and was stored at 4°C until use.

A peptide-modified LNP (abbreviation: B002) was prepared in the same manner as that described above, except that Cys-appended cell-penetrating peptide 2 (CMNYHKSNRHN (SEQ ID NO: 55)) having cysteine at its N-terminal was used instead of the Cys-appended cell-penetrating peptide 1. The Cys-appended cell-penetrating peptide 2 has the amino acid sequence obtained by adding cysteine to the N-terminal of the peptide No. 1-8 (SEQ ID NO: 8).

In the same manner as described above, except that no Cys-appended cell-penetrating peptide was used, a LNP (Cys (control)) in which the maleimide groups were bonded with cysteine was prepared.

Accordingly, as siRNA(LUC)-encapsulating LNPs, the following were obtained: a peptide-modified LNP functionalized with Cys-appended cell-penetrating peptide 1 (A001); a peptide-modified LNP functionalized with Cys-appended cell-penetrating peptide 2 (B002); and a control LNP in which cysteines were bonded (Cys (control)).

### [Experimental Example 18: Inhibitory effect of siRNA(LUC)-encapsulated LNPs on luciferase activity in luciferase-expressing cells]

A549-Luc cells and BxPC-3-Luc#2 cells (both obtained from Riken BioResource Research Center) were cultured in RPMI 1640 medium supplemented with 10% FBS and penicillin-streptomycin, and seeded into a Corning 96-well white plate (product number: 3610) at 2,000 cells/0.1 mL/well.

After incubation at 37 °C, 5% CO₂ for 24 h, 10 µL of siRNA(LUC)-encapsulated LNPs (specifically, Cys (control), A001, or B002) was added to each well so as to yield final siRNA concentrations starting at 100 nM with 3-fold serial dilutions. The cells were then incubated for an additional 24 h at 37 °C, 5% CO₂, and luciferase activity in each well was evaluated using the ONE-Glo EX Luciferase Assay System (Promega Corporation, E8110).

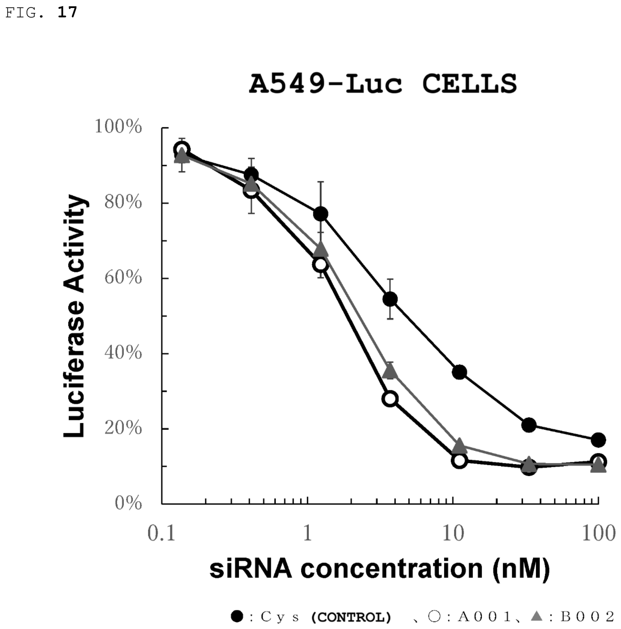

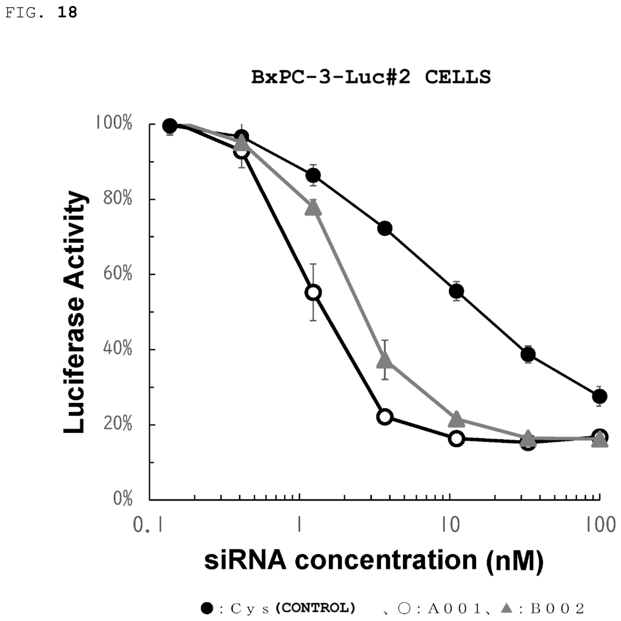

Specifically, after removing the medium from each well, 50 µL of a mixture prepared by combining PBS and ONE-Glo^{®} EX in equal volumes was added to each well, and the plate was shaken at 300 rpm for 3 min in the dark. Next, luminescence of each well was measured with a microplate reader (TECAN Spark) (integration time: 1 second). Luciferase activity for each well was calculated as the ratio of its luminescence intensity to that of wells to which PBS was added in place of the siRNA(LUC)-encapsulated LNP, and plotted against the siRNA concentration. The results for A549-Luc cells are shown in FIG. 17, and those for BxPC-3-Luc#2 cells are shown in FIG. 18 (data points indicate the average of "n" values (n=3) and error bars indicate the standard deviation; provided that when no error bar is visible, the standard deviation is smaller than the symbol).

As shown in FIG. **17** and FIG. **18****,** in both cell types, luciferase activity was suppressed in a concentration-dependent manner with respect to the added siRNA. At the same siRNA concentration, the peptide-modified LNPs (the A001 and the B002) suppressed luciferase activity more strongly than the LNPs (Cys(control)) in which cysteines were bonded in place of the peptide. These results indicate that modifying siRNA (LUC) - encapsulated LNPs with cell-penetrating peptides enables more efficient delivery of the siRNA to the cytosol.

### [Experimental Example 19: Preparation of mRNA-encapsulated Lipid Nanoparticles (LNPs) and Modification thereof with Peptide]

mRNA-encapsulated LNPs were prepared by adding DSG-PEG(2000)-maleimide to Lipid Nanoparticle (LNP-102) Exploration Kit in the same manner as in Experimental Example 17. CleanCap FLuc mRNA (TriLink BioTechnologies, L-7602-100) (mRNA(LUC)), which encodes luciferase, was used as the mRNA.

To achieve the mRNA content recommended in the manual, stock solutions of the lipids were added to the mRNA solution in 50 mM sodium acetate (pH 5.0) and rapidly mixed by pipetting for 15 seconds. The prepared sample was dialyzed in the same manner as in Experimental Example 17, and the recovered sample was used as maleimide group-bearing LNPs.

Subsequently, a 10 mM DMSO solution of Cys-appended cell-penetrating peptide 1 (5 molar equivalents relative to the maleimide groups) was added, and the mixture was incubated for 60 minutes at room temperature. After that, a fivefold molar amount of cysteine (10 mM in PBS) was further added and incubated for 15 minutes at room temperature. After dialysis as described above, the recovered LNP was designated the peptide-modified LNP (abbreviation: AM01), and was stored at 4°C.

In the same manner as described above, except that Cys-appended cell-penetrating peptide 2 was used in place of Cys-appended cell-penetrating peptide 1, a peptide-modified LNP (abbreviation, BM02) was prepared.

A peptide-modified LNP (abbreviation: PM02) was prepared in the same manner as described above, except that Cys-appended cell-penetrating peptide 3 bearing an N-terminal cysteine (CVRVKTYWENH (SEQ ID NO: 56)) was used in place of Cys-appended cell-penetrating peptide 1. Cys-appended cell-penetrating peptide 3 has the amino acid sequence of No. 3-5 (SEQ ID NO: 19) with an N-terminal cysteine appended.

In the same manner as described above, except that no Cys-appended cell-penetrating peptide was used, an LNP in which the maleimide groups were bonded with cysteine (Cys (control)) was prepared.

Accordingly, as mRNA(LUC)-encapsulating LNPs, the following were obtained: a peptide-modified LNP functionalized with Cys-appended cell-penetrating peptide 1 (AM01); a peptide-modified LNP functionalized with Cys-appended cell-penetrating peptide 2 (BM02); a peptide-modified LNP functionalized with Cys-appended cell-penetrating peptide 3 (PM02); and a control LNP in which cysteines were bonded (Cys (control)).

### [Experimental Example 20: Effect of Peptide Modification on Luciferase expression by mRNA(LUC)-encapsulated LNPs]

A549 cells and Panc-1 cells were cultured in RPMI 1640 medium supplemented with 10% FBS and penicillin-streptomycin, and seeded into a Corning 96-well white plate at 10,000 cells/0.1 mL/well.

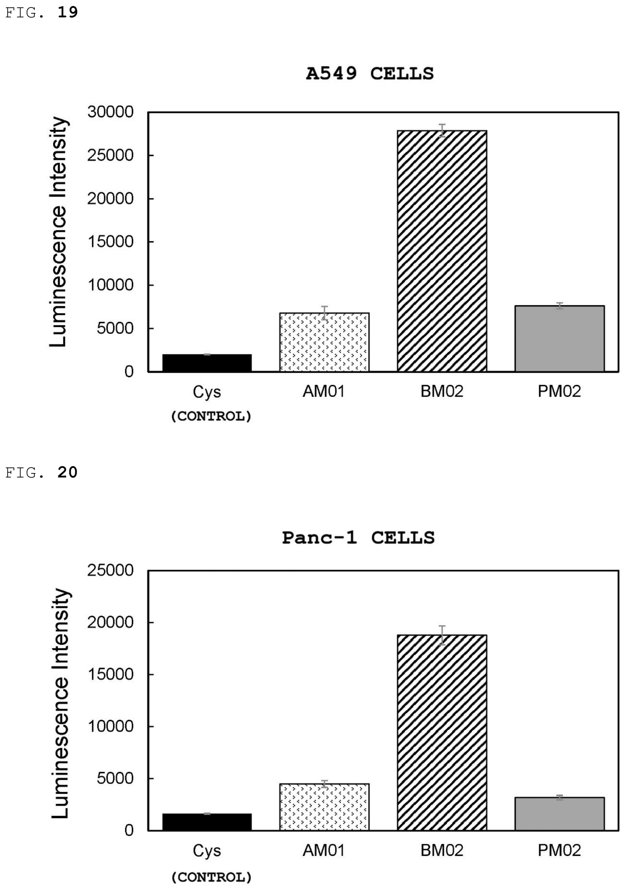

After incubation at 37°C and 5% CO₂ for 24 hours, 10 µL of mRNA(LUC)-encapsulated LNPs (specifically, the Cys(control), AM01, BM02, or PM02) was added to each well to yield a final mRNA dose of 40 ng/well. Incubation was then continued for 24 h at 37°C, 5% CO₂, and luciferase activity in each well was evaluated as in Experimental Example 18 using ONE-Glo EX Luciferase Assay System. The measured luminescence intensities for each well were plotted. Results for A549 cells are shown in FIG. **19****,** and those for Panc-1 cells are shown in FIG. **20** (bars indicate the mean, n = 3; error bars indicate the standard deviation).

As shown in FIG. **19** and FIG. **20****,** for both A549 and Panc-1 cells, stronger luminescence was observed in cells treated with the peptide-modified LNPs (AM01, BM02, and PM02) than in cells treated with LNPs in which cysteines were bonded in place of the peptide (Cys (control)). These results indicate that modifying mRNA(LUC)-encapsulated LNPs with cell-penetrating peptides enables more efficient delivery of the mRNA to the cytosol.

### Industrial Applicability

The cell-penetrating peptides according to embodiments of the present invention may be advantageously used, for example, in the manufacture of DDS systems, pharmaceutical compositions, and the like.

## Claims

1. A peptide or a salt thereof having plasma membrane-penetrating activity, comprising an amino acid sequence X that satisfies the following conditions (1) to (4):
(1) the amino acid sequence X includes at least one amino acid residue selected from R, K, and H, and the total number of these three amino acid residues is 1 to 7;
(2) the amino acid sequence X includes at least one amino acid residue selected from Y, W, and F, and the total number of these three amino acid residues is 1 to 5;
(3) the amino acid sequence X includes at least one amino acid residue selected from L, V, and M, and the total number of these three amino acid residues is 1 to 4; and
(4) the amino acid sequence X has 6 to 15 amino acid residues.

2. The peptide or the salt thereof according to claim 1,
wherein the amino acid sequence X is linear, and within the amino acid sequence X, the number of consecutive occurrences of D, F, L, Q, R, S, V, and Y is 2 or less for each such residue, and the number of consecutive occurrences of the other amino acid residues is 1, or
wherein the amino acid sequence X forms a cyclic structure, and within the amino acid sequence X, the number of consecutive occurrences of F, L, and Y is 2 or less for each such residue, the number of consecutive occurrences of R is 3 or less, and the number of consecutive occurrences of the other amino acid residues is 1.

3. The peptide or the salt thereof according to claim 1,
wherein the amino acid sequence X is linear, and a proportion of the total number of eight amino acid residues E, M, A, L, Q, K, R, and H to the total number of amino acid residues constituting the amino acid sequence X is 25% to 60%, or
wherein the amino acid sequence X forms a cyclic structure via a disulfide bond between two cysteine residues, and a proportion of the total number of eight amino acid residues E, M, A, L, Q, K, R, and H to the total number of amino acid residues constituting the amino acid sequence X excluding the two cysteine residues is 25% to 60%.

4. The peptide or the salt thereof according to claim 1, which exhibits cell-type-selective plasma membrane-penetrating activity.

5. The peptide or the salt thereof according to claim 1, which exhibits selective plasma membrane-penetrating activity toward at least one selected from the group consisting of macrophage cells, cancer cells, neutrophils, mast cells, T cells, B cells, gastrointestinal epithelial cells, the blood-brain barrier, and skin tissue.

6. The peptide or the salt thereof according to claim 1, wherein the number of amino acid residues is 6 to 30.

7. The peptide or the salt thereof according to claim 1, wherein the peptide or the salt thereof has the GRAVY value of from -2.6 to 1.9, and is linear.

8. The peptide or the salt thereof according to claim 1, comprising an amino acid sequence represented by any one of SEQ ID NOs: 1-40 or a homologous sequence thereof,
wherein the homologous sequence comprises 1 to 5 amino acid residue substitutions, insertions, or deletions in the amino acid sequence represented by any one of SEQ ID NOs: 1-40 and has a sequence identity of 60% or more to the amino acid sequence.

9. A drug-peptide conjugate, comprising a drug and the peptide or the salt thereof according to claim 1, the peptide or the salt thereof being bonded to the drug.

10. The drug-peptide conjugate according to claim 9, wherein the drug is a protein or a nucleic acid.

11. Use of the peptide or the salt thereof of claim 1 for drug delivery.

12. A method for screening a peptide having plasma membrane-penetrating activity, comprising the steps of:
(A) preparing a cDNA display library comprising cDNA display molecules;
(B) contacting cells with the cDNA display library and incubating the resulting mixture; and
(C) recovering the cDNA display molecules from intracellular contents of the cells or from a permeate through the cells.

13. The screening method according to claim 12, further comprising preparing a sub-cDNA display library using the recovered cDNA display molecules.

14. The screening method according to claim 12, wherein a selection comprising the steps (A), (B), and (C) in this order is repeated twice or more.
